# EUROPEAN PATENT SPECIFICATION

(11) **EP 2 447 297 B1**
(45) Date of publication and mention of the grant of the patent: **07.10.2015**
(21) Application number: 10792165.2
(22) Date of filing: 24.06.2010
(51) Int. Cl.: C08G 63/02, C07C 43/257, C07C 61/29, C07C 323/62

(54) **NOVEL DEHYDROABIETIC ACID POLYMER**
NEUARTIGES DEHYDRO-ABIETINSÄURE-POLYMER
NOUVEAU POLYMÈRE D'ACIDE DÉHYDROABIÉTIQUE

(30) Priority: 25.06.2009 JP 2009151456
(43) Date of publication of application: 02.05.2012
(73) Proprietor: FUJIFILM Corporation, Tokyo 106-8620 (JP)
(72) Inventor: SAKUMA, Toshimitsu, Ashigarakami-gun Kanagawa 258-8577 (JP); SATO, Kozo, Ashigarakami-gun Kanagawa 258-8577 (JP)
(74) Representative: Hoffmann Eitle
(86) International application number: PCT/JP2010/060758
(87) International publication number: WO 2010/150847

(56) References cited:
- JP-A- 4 153 092
- JP-A- 59 022 919
- JP-A- 2007 292 815
- US-A- 3 835 182

## Description

### [Technical Field]

The present invention relates to a novel dehydroabietic acid polymer, and more specifically, the present invention relates to a novel dehydroabietic acid polymer obtained by using a dehydroabietic acid that is one of the constituent components included in rosin, and a composite material containing the polymer.

### [Background Art]

In recent years, from the viewpoint of global environment protection, reduced dependence on oil as a resource has been investigated, and various natural resources have attracted great attention. Similarly, in the field of plastics, reduction of oil-dependency has been examined, and as a result, for example, polylactic acid that uses lactic acid obtained by the fermentation of glucose as a raw material has been widely used for packaging materials and the like.

According to "Polylactic Acids: Foundation and Application of Plastics Derived from Plants", written by Hideto Tsuji, published by Yoneda Shuppan, 2008, polylactic acids have an excellent transparency, but have low heat resistance, and therefore, the application of polylactic acid to molded products made by injection molding or the like is limited only to a use that does not include exposure to a high temperature.

Further, besides polylactic acid, as is shown in the "Handbook of Polyester Resin" written by Eiichiro Takiyama, published by Nikkan Kogyo Shimbun, Ltd., 1988 and in the "Handbook of Polycarbonate Resin" written by Seiichi Honma, published by Nikkan Kogyo Shimbun, Ltd., 1992, PET (polyethylene terephthalate) and PC (polycarbonate) are caused hydrolysis in a high temperature and high humidity environment or in an acidic or alkaline environment and, as a result, exhibit low moisture resistance. Therefore, improvement thereof is required.

Meanwhile, rosin that can be obtained from pine tree is known as one of important bio-based industrial raw materials. Rosin is composed of various carboxylic acids, and among the carboxylic acids, it is known that abietic acid is utilized in polymer materials (see, for example, Japanese Patent Application Laid-Open (JP-A) Nos. 2008-274150 and 6-87946).

For example, JP-A Nos. 2008-274150 and 6-87946 disclose a technology of preparing a rosin-modified phenol resin or a rosin-modified epoxy resin by modifying a terminal portion of a phenol resin or an epoxy resin with abietic acid and using these resins as an additive for a coating material or the like. However, since these resins consist of a phenol resin or an epoxy resin as a main skeleton thereof, these materials depend on oil and, therefore, they are not environmentally friendly enough.

Further, a polymer obtained by the polymerization of abietic acid and a polyhydric alcohol is also known (see, for example, JP-A No. 6-33395). However, since the polymer described in JP-A No. 6-33395 polymerizes randomly and complicatedly, the polymer cannot form a linear polymer having a high molecular weight. Accordingly, such a polymer cannot be utilized for industrial use as a molded article or the like.

US-A-3,835,182 discloses reacting ethylene and dehydroabietic acid in the presence of palladium (II) acetate to form mixed dimeric basic acids. These acids are useful in the preparation of modified styrenated laminating polyesters.

### SUMMARY OF INVENTION

### [Technical Problem]

An object of the present invention is to provide a novel dehydroabietic acid polymer that is able to use a raw material derived from rosin, which is a natural product, and this polymer has high heat resistance and high moisture and water resistance.

Another object of the present invention is to provide a composite material containing the novel dehydroabietic acid polymer.

### [Solution to Problem]

According to a first aspect, the present invention provides a dehydroabietic acid polymer having a weight average molecular weight of from 5,000 to 500,000 and comprising a repeating unit represented by the following Formula (II): 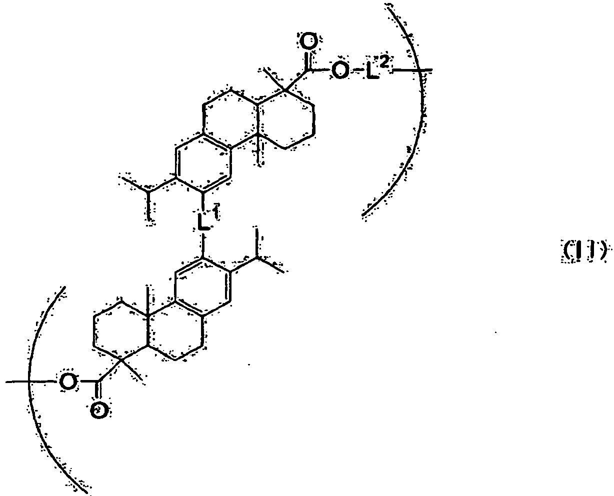 wherein L¹ represents a single bond, -O-, -S- or -CH₂-; and L² represents an alkylene group or an arylene group.

According to a second aspect, the present invention provides a dehydroabietic acid polymer having a weight average molecular weight of from 5,000 to 500,000 and comprising a repeating unit represented by the following Formula (III): 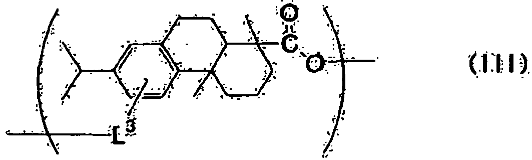 wherein L³ represents a single bond, -(CH₂)₄-, -CO(CH₂)₃-, -(CH₂)₃CO₂-C₆H₄-C(CH₃)₂-C₆H₄- or -CO(CH₂)₂CO₂-C₆H₄-C(CH₃)₂-C₆H₄-.

According to a third aspect, the present invention provides a composite material comprising the dehydroabietic acid polymer in accordance with the above first aspect or second aspect.

According to a fourth aspect, the present invention provides a dehydroabietic acid derivative comprising a compound represented by the following Formula (IV): 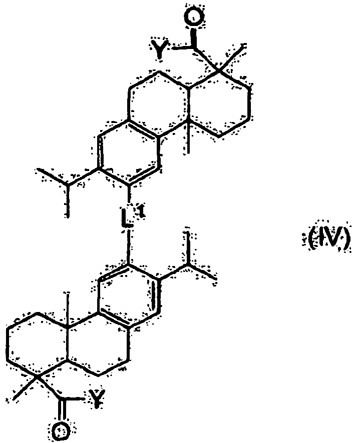 wherein L¹ represents a single bond, -O-, -S- or -CH₂-; Y represents -OH, -OR, -OCOR, -OCOOR, or -OSO₂R; and R represents an alkyl group or an aryl group.

### [Advantageous Effects of Invention]

According to the present invention, a novel dehydroabietic acid polymer is able to use a raw material derived from rosin and has high heat resistance and high moisture and water resistance.

Further, according to present invention, a composite material containing the novel dehydroabietic acid polymer may be provided.

### BRIEF DESCRIPTION OF DRAWINGS

Fig. 1 shows a ¹H-NMR spectrum of compound (4-I) used in Example 4.
Fig. 2 shows a ¹H-NMR spectrum of the dehydroabietic acid polymer obtained in Example 4.

### DESCRIPTION OF EMBODIMENTS

### [Dehydroabietic Acid Polymer]

Herein below, the dehydroabietic acid polymer of the present invention is described.

The dehydroabietic acid polymer of the present invention is a polymer having a repeating unit containing a dehydroabietic acid skeleton.

The dehydroabietic acid polymer of the present invention exhibits high heat resistance and high moisture and water resistance. Further, the dehydroabietic acid that is the raw material of the dehydroabietic acid polymer of the present invention can be obtained from rosin derived from pine tree.

Accordingly, the polymer of the present invention can be provided as a novel bio-based polymer which is superior to conventional bio-based polymers such as polylactic acid in terms of heat resistance and moisture and water resistance.

Moreover, the dehydroabietic acid polymer of the present invention can be utilized for various applications in various forms, for example, in the form of a sheet, a film, a fiber, a molded material, or the like, by putting the characteristics of high heat resistance and high moisture and water resistance into practical use.

The dehydroabietic acid polymer of the present invention is described in detail.

The dehydroabietic acid polymer of the present invention is a homopolymer obtained by the polymerization using dehydroabetic acid represented by the following Formula (A) or a derivative thereof as a raw material monomer, or a copolymer obtained by the polymerization using the monomer represented by the following Formula (A) or a derivative thereof and other monomer, and has a repeating unit containing a dehydroabietic acid skeleton in the molecular structure.

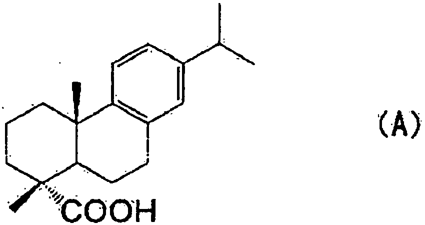

Here, the "dehydroabietic acid skeleton" in the present invention means a skeleton represented by the following Formula (B), which is derived from the above dehydroabietic acid. 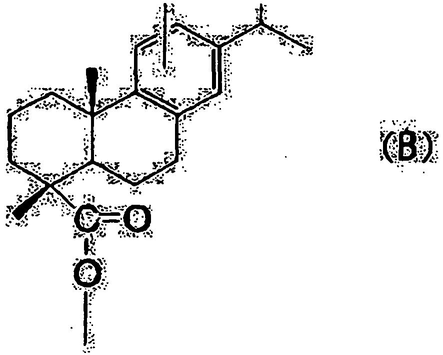 The weight average molecular weight of the dehydroabietic acid polymer of the present invention is from 5,000 to 500,000, and more preferably from 10,000 to 200,000. When the weight average molecular weight is within this range, the dehydroabietic acid polymer has excellent properties in terms of molding and the like, and becomes satisfactory in view of industrial use.

Here, the weight average molecular weight in the present invention is a value obtained by the measurement of molecular weight (in terms of polystyrene) by gel permeation chromatography (GPC).

The dehydroabietic acid polymer of the present invention has moldability and also has excellent heat resistance and excellent moisture and water resistance. The reason for this is considered as follows: namely, the tricyclic portion of the dehydroabietic acid skeleton (the tricyclic portion in the structural formula shown below) is essentially heat-stable and highly hydrophobic, and further, an isopropyl group and a methyl group on the tricyclic portion increase the hydrophobility, and therefore, the dehydroabietic acid polymer of the present invention has the above characteristics.

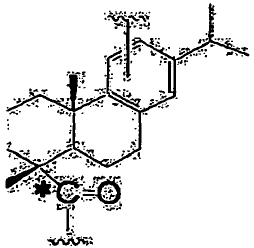

Moreover, the ester structure at the 18th position (*) of the dehydroabietic acid skeleton is extremely stable and has excellent resistance toward hydrolysis, and thus, the excellent moisture and water resistance of the dehydroabietic acid polymer of the present invention was attained.

As described above, conventional bio-based polymers obtained by using biomass resources generally have problems in that they are inferior in heat resistance or moisture and water resistance; however, the dehydroabietic acid polymer of the present invention exhibits excellent heat resistance and excellent moisture and water resistance, even though the dehydroabietic acid polymer can also be produced by using a raw material derived from biomass resources.

The dehydroabietic acid polymer of the present invention also includes a derivative of a dehydroabietic acid polymer which is obtained by further subjecting a polymer having a repeating unit containing a dehydroabietic acid skeleton to a chemical treatment or the like.

In a preferable embodiment of the dehydroabietic acid polymer of the present invention, a dimer structure which is formed by bonding two dehydroabietic acid skeletons directly or through a linking group is contained in the repeating unit. For example, this dimer structure is represented by the skeleton represented by the following Formula (C).

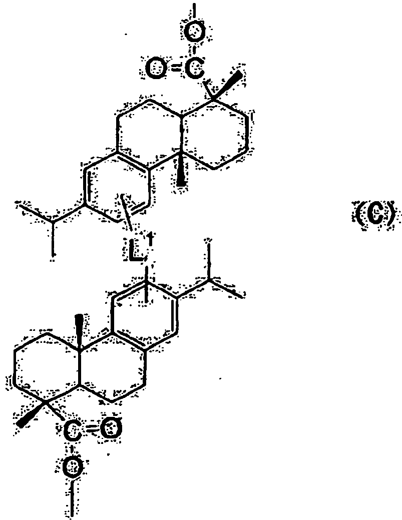

In Formula (C), L¹ represents a single bond, -O-, -S- or -CH₂-.

The dehydroabietic acid polymer of the present invention is preferably a polyester polymer obtained by using a dehydroabietic acid derivative and a diol compound.

In a preferred specific embodiment in a case in which the dehydroabietic acid polymer of the present invention is a polyester polymer, the dehydroabietic acid polymer is a polymer having a repeating unit represented by the following Formula (II).

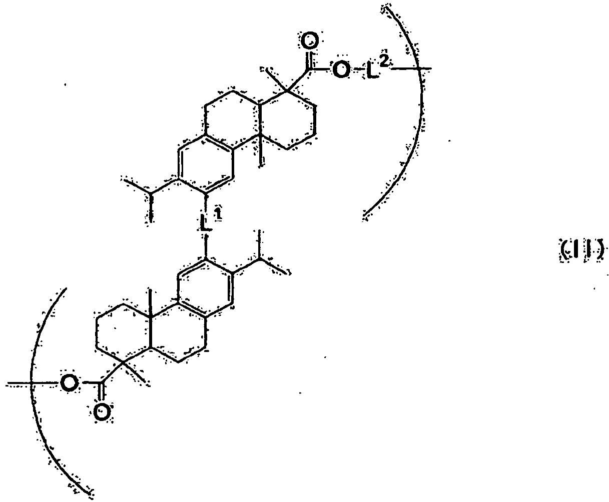

In Formula (II), L¹ represents a single bond, -O-, -S- or -CH₂-; and L² represents an alkylene group or an arylene group.

The alkylene group represented by L² preferably has from 1 to 20 carbon atoms, and particularly preferably from 2 to 12 carbon atoms. The alkylene group represented by L² may be straight chain, branched, or cyclic, and may further have a substituent.

The alkylene group represented by L² may have a structure in which at least one carbon atom in the molecular chain is replaced with an oxygen atom.

Specific examples of the alkylene group represented by L² include -(CH₂)₂-, -(CH₂)₃-, -(CH₂)₄-, -(CH₂)₆-, -(-CH₂)₈-, -(CH₂)₁₀-, -CH₂CH₂OCH₂CH₂-, -CH₂CH₂(OCH₂CH₂)₂-, -CH₂CH₂(OCH₂CH₂)₃-, and -CH₂CH₂OC₆H₄OCH₂CH₂-.

The arylene group represented by L² preferably has from 6 to 20 carbon atoms, and particularly preferably from 6 to 15 carbon atoms. The arylene group represented by L² may be a monocycle or a condensed ring, and may further have a substituent.

Specific examples of the arylene group represented by L² include -C₆H₄- and -C₆H₄-C(CH₃)₂-C₆H₄-.

L² preferably represents -(CH₂)₃-, -(CH₂)₁₀-, or -CH₂CH₂(OCH₂CH₂)₃-.

Examples of the polyester polymer include: in Formula (II), a polyester polymer having a structure in which L¹ represents a single bond, and L² represents -(-CH₂)₂-, -(CH₂)₃-, -CH₂CH(CH₃)-, -(CH₂)₄-, -(CH₂)₆-, -(CH₂)₈-, -(CH₂)₁₀-, -(CH₂)₁₂-, -CH₂CH₂OCH₂CH₂-, -CH₂CH₂(OCH₂CH₂)₂-, -CH₂CH₂(OCH₂CH₂)₃-, -CH₂CH₂OC₆H₄OCH₂CH₂-, -C₆H₄-, or -C₆H₄C(CH₃)₂C₆H₄-; a polyester polymer having a structure in which L¹ represents an oxygen atom, and L² represents -(CH₂)₂-, -(CH₂)₃-, -CH₂CH(CH₃)-, -(CH₂)₄-, -(CH₂)₆-, -(CH₂)₈-, -(CH₂)₁₀-, -(CH₂)₁₂-, -CH₂CH₂OCH₂CH₂-, -CH₂CH₂(OCH₂CH₂)₂-, -CH₂CH₂(OCH₂CH₂)₃-, -CH₂CH₂OC₆H₄OCH₂CH₂-, -C₆H₄-, or -C₆H₄C(CH₃)₂C₆H₄-; a polyester polymer having a structure in which L¹ represents a sulfur atom, and L² represents -(CH₂)₂-, -(CH₂)₃-, -CH₂CH(CH₃)-, -(CH₂)₄-, -(CH₂)₆-, -(CH₂)₈-, -(CH₂)₁₀-, -(CH₂)₁₂-, -CH₂CH₂OCH₂CH₂-, -CH₂CH₂(OCH₂CH₂)₂-, -CH₂CH₂(OCH₂CH₂)₃-, -CH₂CH₂OC₆H₄OCH₂CH₂-, -C₆H₄-, or -C₆H₄C(CH₃)₂C₆H₄-; a polyester polymer having a structure in which L¹ represents -CH₂-, and L² represents -(CH₂)₂-, -(CH₂)₃-, -CH₂CH(CH₃)-, -(CH₂)₄-, -(CH₂)₆-, -(CH₂)₈-, -(CH₂)₁₀-, -(CH₂)₁₂-, -CH₂CH₂OCH₂CH₂-, -CH₂CH₂(OCH₂CH₂)₂-, -CH₂CH₂(OCH₂CH₂)₃-, -CH₂CH₂OC₆H₄OCH₂CH₂-, -C₆H₄-, or -C₆H₄C(CH₃)₂C₆H₄-;

Among them, a polyester polymer having a structure in which, in Formula (II), L¹ represents a single bond, and L² represents -(CH₂)₂-, -(CH₂)₃-, -CH₂CH(CH₃)-, -(CH₂)₄-, -(CH₂)₆-, -(CH₂)₈-, -(CH₂)₁₀-, -CH₂CH₂(OCH₂CH₂)₃-, -CH₂CH₂OC₆H₄OCH₂CH₂-, -C₆H₄-, or -C₆H₄C(CH₃)₂C₆H₄-;
a polyester polymer having a structure in which L¹ represents an oxygen atom, and L² represents -(CH₂)₂-, -(CH₂)₃-, -CH₂CH(CH₃)-, -(CH₂)₄-, -(CH₂)₆-, -(CH₂)₈-, -(CH₂)₁₀-, -CH₂CH₂(OCH₂CH₂)₃-, -CH₂CH₂OC₆H₄OCH₂CH₂-, -C₆H₄-, or -C₆H₄C(CH₃)₂C₆H₄-;
a polyester polymer having a structure in which L¹ represents a sulfur atom, and L² represents -(CH₂)₂-, -(CH₂)₃-, -CH₂CH(CH₃)-, -(CH₂)₄-, -(CH₂)₆-, -(CH₂)₈-, -(CH₂)₁₀-, -CH₂CH₂(OCH₂CH₂)₃-, -CH₂CH₂OC₆H₄OCH₂CH₂-, -C₆H₄-, or -C₆H₄C(CH₃)₂C₆H₄-; a polyester polymer having a structure in which L¹ represents -CH₂-, and L² represents -(CH₂)₂-, -(CH₂)₃-, -CH₂CH(CH₃)-, -(CH₂)₄-, -(CH₂)₆-, -(CH₂)₈-, -(CH₂)₁₀-, -CH₂CH₂(OCH₂CH₂)₃-, -CH₂CH₂OC₆H₄OCH₂CH₂-, -C₆H₄-, or -C₆H₄C(CH₃)₂C₆H₄-;

Particularly preferable polyester polymers are a polyester polymer having a structure, in which, in Formula (II), L¹ represents a single bond, and L² represents -(CH₂)₃-, -(CH₂)₁₀-, or -CH₂CH₂(OCH₂CH₂)₃-;
a polyester polymer having a structure, in which L¹ represents an oxygen atom, and L² represents -(CH₂)₃-, -(CH₂)₁₀-, or -CH₂CH₂(OCH₂CH₂)₃-;
a polyester polymer having a structure, in which L¹ represents a sulfur atom, and L² represents -(CH₂)₃-, -(CH₂)₁₀-, or -CH₂CH₂(OCH₂CH₂)₃-; and
a polyester polymer having a structure, in which L¹ represents -CH₂-, and L² represents -(CH₂)₃-, -(CH₂)₁₀-, or -CH₂CH₂(OCH₂CH₂)₃-.

The dicarboxylic acid compound or derivative thereof which can be used as the raw material of the dehydroabietic acid polymer, that is a polyester polymer, may be a compound represented by the following Formula (IV) or a derivative thereof.

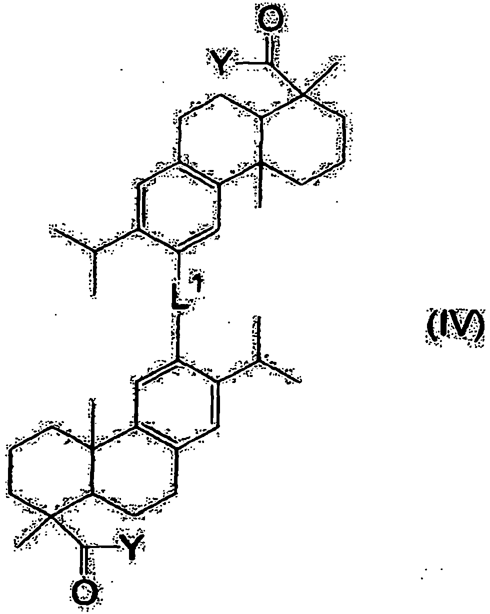

In Formula (IV), L¹ represents a single bond, -O-, -S- or -CH₂-; Y represents -OH, -OR, -OCOR, -OCOOR, or -OSO₂R; and R represents an alkyl group or an aryl group.

Examples of the diol compound, which is represented by the following Formula: HO-L²-OH, include aliphatic diols such as ethylene glycol, 1,2-propanediol, 1,3-propanediol, 1,4-butanediol, 1,6-hexanediol, 1,8-octanediol, 1,10-decanediol, 1,12-dodecanediol, diethylene glycol, triethylene glycol, tetraethylene glycol, or 1,4-bis(2-hydroxyethoxy)benzene; and aromatic diols such as hydroquinone, 2,2-bis(4-hydroxyphenyl)propane, and from the viewpoint of not lowering the degree of plant composition, 1,3-propanediol, 1,10-decanediol, and the like are more preferable.

Further, another example of the dehydroabietic acid polymer of the present invention is a polymer including a repeating unit represented by the following Formula (III). This polymer is a single molecule self-condensation type polymer.

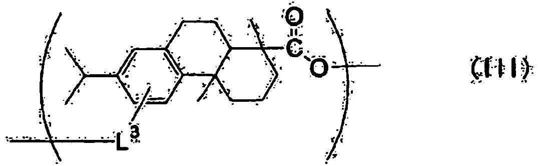

In Formula (III), L³ represents a single bond, -(CH₂)₄-, -CO(CH₂)₃-, -(CH₂)₃CO₂-C₆H₄-C(CH₃)₂-C₆H₄- or -CO(CH₂)₂CO₂-C₆H₄-C(CH₃)₂-C₆H₄-.

Examples of a monomer (self-condensation type monomer) that is used for synthesizing the polymer including a repeating unit represented by Formula (III) include a compound represented by the following Formula (V) and a derivative thereof.

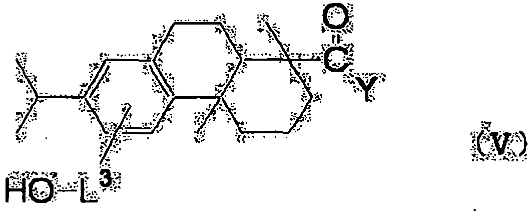

In Formula (V), L³ represents a single bond, -(CH₂)₄-, -CO(CH₂)₃-, -(CH₂)₃CO₂-C₆H₄-C(CH₃)₂-C₆H₄- or -CO(CH₂)₂CO₂-C₆H₄-C(CH₃)₂-C₆H₄-. Y represents a chlorine atom, -OH, -OR, -OCOR, -OCOOR, or -OSO₂R, and R represents an alkyl group or an aryl group.

### [Method for Producing Dehydroabietic Acid Polymer]

The method for producing the dehydroabietic acid polymer of the present invention is described.

The dehydroabietic acid polymer of the present invention is not particularly limited as long as the polymer is, as described above, a homopolymer obtained by the polymerization using dehydroabetic acid represented by Formula (A) above or a derivative thereof as a raw material monomer, or a copolymer obtained by the polymerization using dehydroabetic acid derivatives represented by Formula (A) above and other monomer, and has a repeating unit containing a dehydroabietic acid skeleton in the molecular structure.

The dehydroabietic acid used for the production of the dehydroabietic acid polymer can be obtained from rosin.

Rosin is a resin component which can be obtained from pine tree, and is classified into three types: "gum rosin", "tall rosin", and "wood rosin", according to the extraction method. The components included in rosin are different depending on their extraction method or the growing district of pines, but generally, the components are a mixture of diterpene resin acids, such as abietic acid (1), neoabietic acid (2), palustric acid (3), levopimaric acid (4), dehydroabietic acid (5), pimaric acid (6), and isopimaric acid (7), the structures of which are shown below.

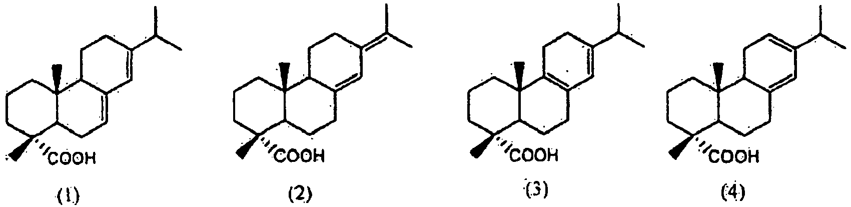

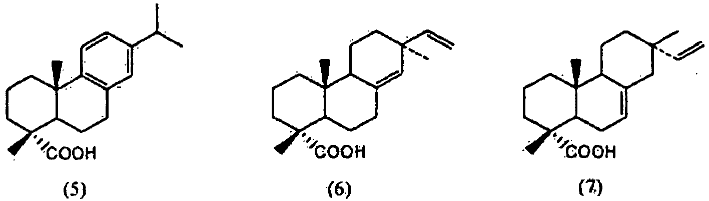

Among these diterpene resin acids, the compounds represented by (1) to (4) may cause disproportionation by a heat treatment in the present of a certain kind of metal catalyst, to be modified into dehydroabietic acid (5) and dihydroabietic acid (8) having the following structure.

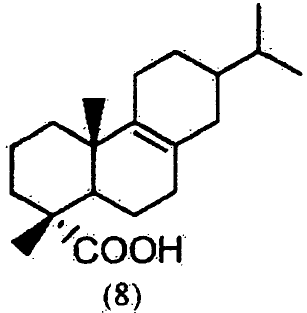

Namely, the dehydroabietic acid (5) which is necessary for carrying out the synthesis of the dehydroabietic acid polymer of the present invention can be obtained relatively easily by subjecting rosin, which is a mixture of various resin acids, to an appropriate chemical treatment, and can also be industrially produced at low costs. Further, the dihydroabietic acid (8) and the dehydroabietic acid (5) can be readily separated by a known method.

The dehydroabietic acid polymer of the present invention can be synthesized, for example, according to the following synthetic route 1 or 2. Here, the synthetic routes 1 and 2 are examples of a synthetic route for synthesizing the polymer having a repeating unit represented by Formula (II) above, which is a polyester polymer, as the dehydroabietic acid polymer of the present invention.

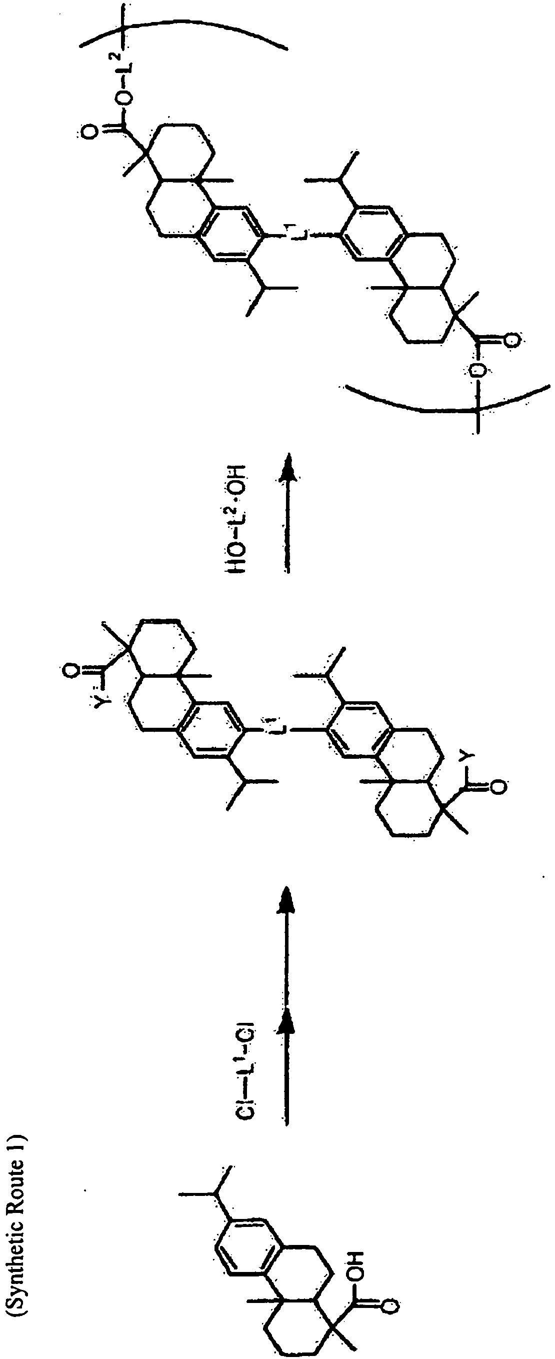

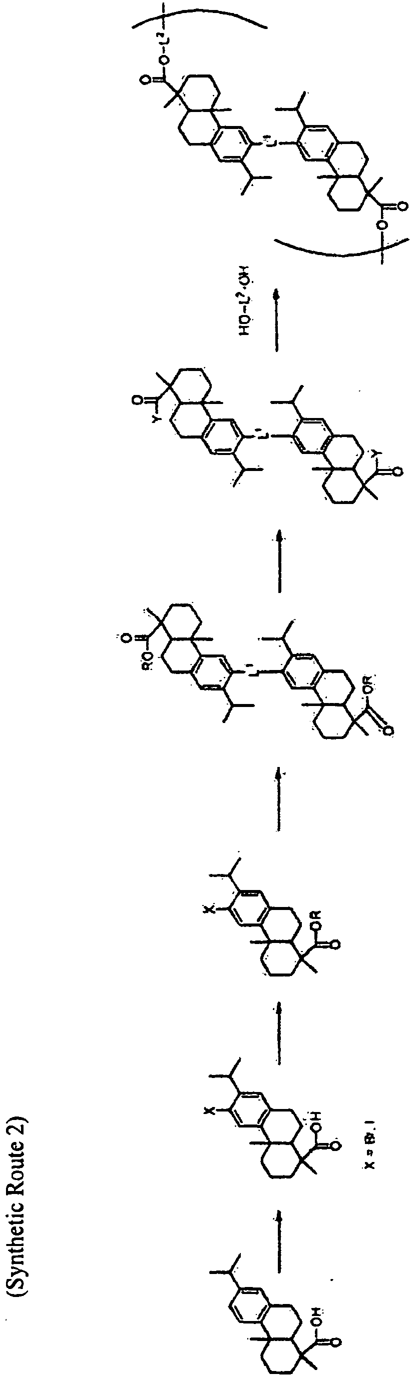

In the above synthetic route 1, L¹, and Y are those explained in Formula (IV), respectively.

In the above synthetic route 2, L¹, R, and Y are those explained in Formula (IV), respectively.

Herein below, the process (the process shown at the right hand end in the synthetic routes 1 and 2) of synthesizing the polyester polymer, which is the final product, from the compound represented by Formula (IV) above or a derivative thereof and a diol compound, in the synthetic routes 1 and 2, is described in detail. Note that, details on the synthesis examples of the polyester polymer according to the synthetic routes 1 and 2 are further explained specifically in the Examples described below.

In the synthetic routes 1 and 2, in the process of synthesizing the polymer (polyester polymer) having a repeating unit represented by Formula (II), the polyester polymer can be synthesized by polycondensation reaction between a diol compound (preferably, an aliphatic diol compound) and the dicarboxylic acid chloride or diester included in the compound represented by Formula (IV).

Specific examples of the synthetic method include methods (for example, a melt polymerization method such as a transesterification method, a direct esterification method, or an acid chloride method, a low temperature solution polymerization method, a high temperature solution polycondensation method, an interfacial polycondensation method, or the like) described, for example, in Shin Kobunshi Jikkengaku (New Polymer Experimentology) 3, Kobunshi no Gosei Hanno (Synthesis and Reaction of Polymer) (2), pages 78 to 95, Kyoritsu Press (1996); and particularly, an acid chloride method or an interfacial polycondensation method is preferably used in the present invention.

The transesterification method is a method in which the aliphatic diol compound and the dicarboxylic acid ester are heated in the molten state or in the solution state, as necessary in the presence of a catalyst, thereby allowing to react dealcoholation polycondensation, to synthesize the polyester.

The direct esterification method is a method in which the aliphatic diol compound and the dicarboxylic acid compound are heated in the molten state or in the solution state, in the presence of a catalyst, thereby allowing to react dehydration polycondensation, to synthesize the polyester.

The acid chloride method is a method in which the aliphatic diol compound and the dicarboxylic acid chloride compound are heated in the molten state or in the solution state, as necessary in the presence of a catalyst, thereby allowing to react HCl-elimination polycondensation, to synthesize the polyester.

The interfacial polymerization method is a method including dissolving the aliphatic diol compound in water, dissolving the dicarboxylic acid compound in an organic solvent, and allowing to react polycondensation at the water/ organic solvent interface using a phase transfer catalyst, thereby synthesizing the polyester.

Further, in a case in which the dehydroabietic acid polymer of the present invention is synthesized as a polymer having a repeating unit represented by Formula (III) above, the polymer can be synthesized by using a self-condensation type monomer derived from dehydroabietic acid, and allowing this monomer to react self-condensation. An example of a synthetic route for synthesizing the polymer having a repeating unit represented by Formula (III) above, that is a polyester polymer, as the dehydroabietic acid polymer of the present invention, is the following synthetic route 3.

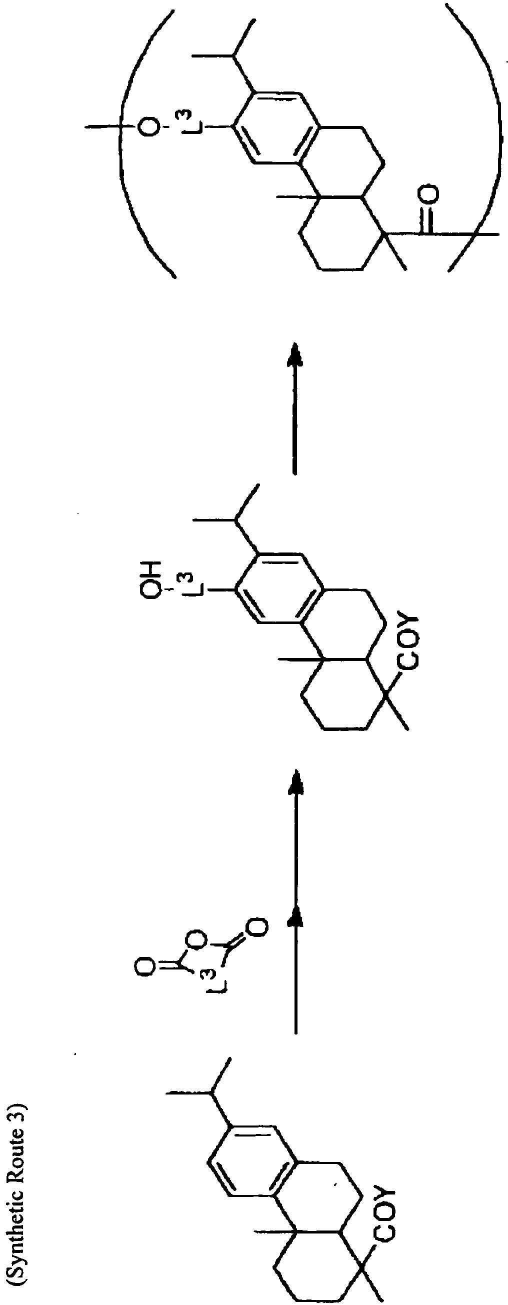

In the above synthetic route 3, L³ and Y are those explained in Formula (V), respectively.

Details on the synthesis example of the polymer including a repeating unit represented by Formula (III) are further explained specifically in the Examples described below.

The dehydroabietic acid polymer of the present invention as described above can be used singly as a polymer material. Alternatively, the dehydroabietic acid polymer of the present invention and various materials may be mixed to produce a composite material.

In the following, the composite material containing the dehydroabietic acid polymer of the present invention is described.

### [Composite Material Containing Dehydroabietic Acid Polymer]

The dehydroabietic acid polymer of the present invention may be mixed with various materials for the purpose of improving the physical properties, to produce a composite material.

In a case in which the dehydroabietic acid polymer is used to produce a composite material, polymer alloying (mixing of different kinds of polymers) and mixing of a filler are especially important, and by carrying out these processes, the impact resistance, heat resistance, durability, moldability, and the like can be improved.

As the polymers used for polymer alloying, two or ore kinds of the dehydroabietic acid polymers of the present invention having different polymer characteristics may be used, or the dehydroabietic acid polymer of the present invention and a polymer other than the dehydroabietic acid polymer may be used in combination.

Examples of the polymer other than the dehydroabietic acid polymer of the present invention, which may be used for polymer alloying, include:
1) olefin-based resins (a homopolymer of α-olefin such as ethylene, propylene, 1-butene, 1-pentene, 1-hexene, or 4-methyl-1-pentene; a homopolymer of cycloolefin such as cyclopentene, cyclohexene, cyclooctene, cyclopentadiene, 1,3-cyclohexadiene, bicyclo[2.2.1]hept-2-ene, tricyclo[4.3.0.1^{2,5}]deca-3,7-diene, or tetracyclo[4.4.0.1^{2,5}.1^{7,10}]dodec-3-ene; a copolymer of α-olefins described above, a copolymer of α-olefin and other monomer capable of copolymerization, for example, vinyl acetate, maleic acid, vinyl alcohol, methacrylic acid, methyl methacrylate, ethyl methacrylate, or the like; or the like);
2) polyester-based resins (a copolymer of a dicarboxylic acid monomer, such as terephthalic acid, isophthalic acid, 2,6-naphthalenedicarboxylic acid, 1,4-naphthalenedicarboxylic acid, succinic acid, adipic acid, or sebacic acid, and a diol or polyhydric alcohol monomer, such as ethylene glycol, propylene glycol, 1,4-butylene glycol, 1,4-cyclohexanedimethanol, diethylene glycol, triethylene glycol, polypropylene glycol, polyoxytetramethylene glycol, an alkylene oxide adduct of a bisphenol compound or a derivative thereof, trimethylolpropane, glycerin, or pentaerythritol; a polycondensation product of hydroxycarboxylic acid or the like, such as lactic acid, β-hydroxybutyric acid, p-hydroxybenzoic acid, or 2,6-hydroxynaphthoic acid; or the like);
3) polyamide-based resins (a polymer having an acid-amide bond in the chain thereof, which is obtained by polycondensation of a lactam having a three or more-membered ring structure, or an ω-amino acid or dibasic acid capable of polymerization with a diamine or the like, specifically, a polymer of ε-caprolactam, aminocaproic acid, enantlactam, 7-aminoheptanoic acid, 11-aminoundecanoic acid, 9-aminononanoic acid, α-pyrrolidone, α-piperidone, or the like; or a polymer obtained by polycondensation of a diamine, such as hexamethylenediamine, nonamethylenediamine, undecamethylenediamine, dodecamethylenediamine, or meta-xylenediamine, with a dicarboxylic acid, such as terephthalic acid, isophthalic acid, adipic acid, sebacic acid, dodecane dibasic acid, or glutaric acid, or a copolymer thereof, for example, nylon-4, nylon-6, nylon-7, nylon-8, nylon-11, nylon-12, nylon-6,6, nylon-6,10, nylon-6,11, nylon-6,12, nylon-6T, a nylon-6/ nylon-6,6 copolymer, a nylon-6/ nylon-12 copolymer, a nylon-6/ nylon-6T copolymer, a nylon-6I/ nylon-6T copolymer, or the like);
4) rubbers and elastomers (natural rubber, isoprene rubber, butadiene rubber, 1,2-polybutadiene rubber, styrene-butadiene rubber, chloroprene rubber, nitrile rubber, butyl rubber, ethylene-propylene rubber, chlorosulfonated polyethylene, acrylic rubber, epichlorohydrin rubber, polysulfide rubber, silicone rubber, fluorine-containing rubber, urethane rubber, or the like);
and, in addition to the above polymers, resins such as a polycarbonate-based resin, an acryl-based resin, a urethane-based resin, polyvinyl alcohol, a vinyl chloride-based resin, a styrene-based resin, polyacrylonitrile, polyvinylidene chloride, a fluororesin, polyacetal, polysulfone, ABS resin, or polyetheretherketone.

Among the above polymers, which may be used for polymer alloying, polylactic acid, poly(β-hydroxylactic acid), polybutylene succinate, or the like is preferably used from the viewpoint of not lowering the degree of plant composition.

Polymer alloying is generally performed by melt kneading; however, in a case in which phase separation occurs when performing simple kneading, a homogeneous phase is formed by, for example, using a compatibilizing agent, secondarily conducting block polymerization or graft polymerization, or dispersing one of the polymers in a cluster form.

Further, from the viewpoint of performing polymer alloying without impairing the characteristics of the dehydroabietic acid polymer of the present invention, the content ratio (on the basis of mass) of the dehydroabietic acid polymer of the present invention in the polymer alloy is preferably from 20% to 100%, and more preferably from 50% to 100%.

Moreover, the dehydroabietic acid polymer of the present invention can be improved to have desired polymer physical properties by mixing with various kinds of filler. In particular, mixing of filler is effective in improving the heat resistance, durability, and impact resistance.

Either an inorganic filler or an organic filler may be used as the filler.

Examples of a useful inorganic filler include fibrous inorganic fillers such as glass fiber, carbon fiber, graphite fiber, metallic fiber, potassium titanate whisker, aluminium borate whisker, magnesium-based whisker, silicon-based whisker, wollastonite, sepiolite, slug fiber, zonolite, ellestadite, gypsum fiber, silica fiber, silica alumina fiber, zirconia fiber, boron nitride fiber, silicon nitride fiber, or boron fiber; and plate-like or granular inorganic fillers such as glass flake, non-swelling mica, fullerene, carbon nanotube, carbon black, graphite, metallic foil, ceramic beads, talc, clay, mica, sericite, zeolite, bentonite, dolomite, kaolin, fine powdered silicic acid, feldspar powder, potassium titanate, shirasu balloon, calcium carbonate, magnesium carbonate, barium sulfate, calcium oxide, aluminium oxide, titanium oxide, magnesium oxide, aluminium silicate, silicon oxide, aluminium hydroxide, magnesium hydroxide, gypsum, novaculite, dawsonite, or terra alba.

Examples of a useful organic filler include synthetic fibers such as cellulose nanofiber, polyester fiber, nylon fiber, acrylic fiber, regenerated cellulose fiber, acetate fiber, or aramid fiber; natural fibers of kenaf, rami, cotton, jute, hemp, sisal, Manila hemp; flax, linen, silk, wool, or the like; fibrous organic fillers obtained from microcrystalline cellulose, sugar cane, wood pulp, wastepaper, used paper, or the like; and granular organic fillers such as organic pigments.

In most cases, a flame retardant is mixed with the dehydroabietic acid polymer of the present invention to produce a composite material, which is applied as a practical product.

A flame retardant is a material that makes a polymer material hard to burn or inhibits the spread of flame.

A halogen-based compound (a bromine or chlorine compound) or a phosphorus-based compound (an aromatic phosphate ester or the like) is mainly used as the flame retardant. However, these flame retardants generate substances which are toxic to the human body, or produce environmentally hazardous substances by fire, and therefore, improvement is required. From the viewpoints described above, aluminium hydroxide or magnesium hydroxide, each of which has attracted attention as a material superior in flame retarding effect and environmental safety, is preferably used as the flame retardant that is used in combination with the dehydroabietic acid polymer of the present invention.

A material (a flame retarding aid), which enhances the flame retardency when used in combination with a flame retardant or forms a carbonized membrane on a resin surface to suppress the spread of fire, is also useful for the composite material containing the dehydroabietic acid polymer of the present invention. Specifically, an antimony compound as an inorganic material, or an organic aromatic compound (a phenol derivative or the like) is preferably used.

Further, to the dehydroabietic acid polymer of the present invention, in addition to the above substances, additives that are generally used, for example, plasticizers, stabilizers, impact resistance enhancers, crystal nucleating agents, slipping agents, antistatic agents, surfactants, pigments, dyes, fillers, antioxidants, processing aids, ultraviolet absorbents, anti-fog agents, antifungal agents, mildew-proofing agents, or the like, may be added alone or in a combination of two or more kinds of them.

The composite material of the present invention, which is obtained by mixing the materials described above, can be processed (molded) by various methods. As a method for molding, for example, extrusion molding, injection molding, or the like is used. The molded articles obtained as described above are used in applications such as constituent parts of automobile, electric household appliances, electrical and electronic equipments (OA or media related equipments, optical instruments, communication equipments, or the like), machine parts, materials for housing and construction, or various vessels such as containers or bottles; however, the present invention is not particularly limited thereto.

The disclosure of Japanese Patent Application No. 2009-151456, filed on June 25, 2009, is incorporated by reference herein in its entirety.

All publications, patent applications, and technical standards mentioned in this specification are herein incorporated by reference to the same extent as if such individual publication, patent application, or technical standard was specifically and individually indicated to be incorporated by reference.

### EXAMPLES

Herein below, the present invention will be specifically described with respect to Examples, but the present invention is not limited to these Examples.

First, the compounds used in the synthesis of the dehydroabietic acid polymers of the present invention were synthesized from dehydroabietic acid as shown in the following synthesis examples [1] to [5].
[1] Synthesis Example of Dicarboxylic Acid Compound Having a Structure Represented by Formula (IV) in which L¹ Represents a Single Bond and Y Represents -OH 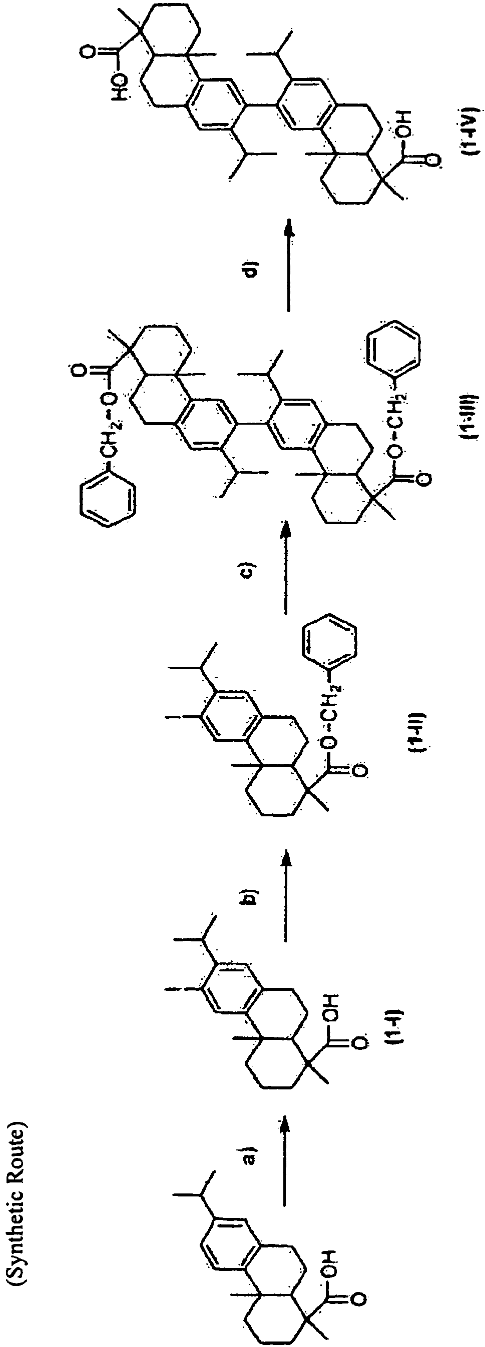
   a) Dehydroabietic acid (135 g, 0.500 mol) was placed in a 1L three-necked flask equipped with a condenser tube, and was dissolved in acetic acid (450 mL). To the reaction system, orthoperiodic acid dihydrate (20.4 g, 0.0895 mol) and iodine (93 g, 0.366 mol) were added. Thereafter, concentrated sulfuric acid (15 mL)/ water (90 mL) was added thereto dropwise, followed by stirring at 60°C for 5 hours. Then, the reaction liquid was left to cool, and then the reaction liquid was poured into water, stirred for one hour, and subjected to filtration. The resulting residue was washed by pouring methanol over it, to obtain compound (1-I) (128 g, 0.300 mol, 60%).
   b) The compound (1-1) (27.0 g, 63.3 mmol) was placed in a 200 mL three-necked flask equipped with a condenser tube, and was dissolved in N,N-dimethylacetamide (60 mL). To the reaction system, potassium carbonate (11 g, 79.5 mmol) was added, and then benzyl chloride (8.41 g, 66.4 mmol) was added dropwise, followed by stirring at 50°C for 3 hours. The reaction liquid was left to cool, then the reaction liquid was added to water, extracted with ethyl acetate, followed by separating the layers, and then the organic layer was washed with a saturated aqueous solution of sodium chloride, and dried over magnesium sulfate. The solvent was evaporated under reduced pressure, and the resulting concentrate was washed by pouring methanol over it, to obtain compound (1-II) (29.2 g, 56.5 mmol, 89.2%).
   c) The compound (1-II) (21.7 g, 42.0 mmol) was placed in a 500 mL three-necked flask equipped with a condenser tube, and was dissolved in hexamethylphosphoric triamide (200 mL). To the reaction system, dichlorobis(triphenylphosphine)nickel(II) (27.5 g, 42.0 mmol), tricyclohexylphosphine (1.18 g, 4.21 mmol), potassium iodide (7.0 g, 42.1 mmol), and zinc (6.3 g, 96.3 mmol) were added, followed by stirring at 60°C for 5 hours. Then, the reaction liquid was left to cool, and then the reaction liquid was added to dilute hydrochloric acid, extracted with ethyl acetate, and subjected to filtration using celite, followed by separating the layers, and then the organic layer was washed with a saturated aqueous solution of sodium hydrogencarbonate and a saturated aqueous solution of sodium chloride, and subsequently dried over magnesium sulfate. The solvent was evaporated under reduced pressure, and the resulting concentrate was purified by silica gel column chromatography, to obtain compound (1-III) (6.54 g, 8.40 mmol, 40.0%).
   d) The compound (1-III) (2.8 g, 3.59 mmol) was placed in a 200 mL autoclave, and was dissolved in tetrahydrofuran (60 mL). To the reaction system, 10% Pd-C (0.3 g) was added, followed by stirring at 60°C under a hydrogen pressure of 5 MPa for 12 hours. Then, the reaction liquid was left to cool, and the reaction liquid was filtrated, thereby removing the solvent, and then the residue was washed by pouring methanol over it, to obtain compound (1-IV) (2.05 g, 95.0%) that was a dicarboxylic acid compound.
      ¹H-NMR data of the compound (1-IV) are shown below.
      ¹H NMR (300 MHz, CDCl₃) δ 0.90 to 2.40 (m, 42H), 2.49 to 2.77 (m, 2H), 2.81 to 3.09 (m, 4H), 6.88 to 6.98 (m, 2H), 6.98 (s, 2H)
[2] Synthesis Example of Dicarboxylic Acid Compound Having a Structure Represented by Formula (IV) in which L¹ Represents an Oxygen Atom and Y Represents -OH 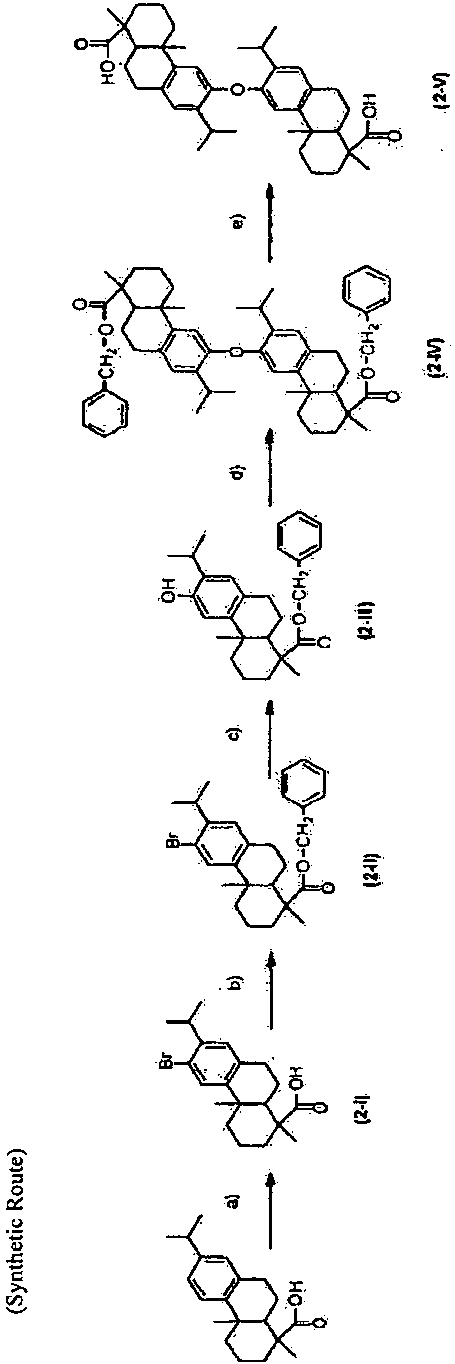
   a) Dehydroabietic acid (90.1 g, 0.300 mol) was placed in a 1L three-necked flask equipped with a condenser tube and was dissolved in acetic acid (500 mL), and nitrogen was blown into the flask at room temperature. Thereafter, bromine (53.0 g, 0.330 mol) was added thereto dropwise, followed by stirring at room temperature for 8 hours. Then, the reaction liquid was poured into water, stirred for one hour, and then subjected to filtration. The resulting residue was washed by pouring methanol over it, to obtain compound (2-1) (61.1 g, 0.161 mol, 53.7%).
   b) The compound (2-1) (7.50 g, 20.0 mmol) was placed in a 200 mL three-necked flask equipped with a condenser tube, and was dissolved in N,N-dimethylacetamide (30 mL). To the reaction system, potassium carbonate (3.28 g, 23.7 mmol) was added, and then benzyl chloride (2.66 g, 21.0 mmol) was added dropwise, followed by stirring at 50°C for 3 hours. Then, the reaction liquid was added to water, extracted with ethyl acetate, followed by separating the layers, and then the organic layer was washed with a saturated aqueous solution of sodium chloride, and dried over magnesium sulfate. The solvent was evaporated under reduced pressure, and the resulting concentrate was washed by pouring methanol over it, to obtain compound (2-II) (7.01 g, 14.9 mmol, 74.5%).
   c) The compound (2-II) (11.7 g, 25.0 mmol) was placed in a 200 mL three-necked flask equipped with a reflux tube, and was dissolved in 1,4-dioxane (20 mL), and then an aqueous solution (20 mL) obtained by dissolving potassium hydroxide (14.0 g, 250 mmol) was added thereto. To the reaction system, tris(benzylideneacetone)dipalladium(0) (1.14 g, 1.24 mmol) and di-tert-butylphosphino-2',4',6'-triisopropylbiphenyl (1.10 g, 2.59 mmol) were added, followed by refluxing at 100°C for 5 hours. Then, the reaction liquid was left to cool, and then the reaction liquid was added to dilute hydrochloric acid, extracted with ethyl acetate, and subjected to filtration using celite, followed by separating the layers, and then the organic layer was washed with a saturated aqueous solution of sodium hydrogencarbonate and a saturated aqueous solution of sodium chloride, and subsequently dried over magnesium sulfate. The solvent was evaporated under reduced pressure, and the resulting concentrate was purified by silica gel column chromatography, to obtain compound (2-III) (9.22 g, 22.7 mmol, 90.8%).
   d) The compound (2-III) (2.44 g, 6.00 mmol) and the compound (2-II) (2.34 g, 5.00 mmol) were placed in a 100 mL three-necked flask equipped with a condenser tube, and were suspended in toluene (20 mL). To the reaction system, tripotassium phosphate (2.55 g, 12.0 mmol), palladium acetate (0.11 g, 0.5 mmol), and di-tert-butylphosphino-2',4',6'-triisopropylbiphenyl (0.21 g, 0.5 mmol) were added, followed by stirring at 100°C for 5 hours. Then, the reaction liquid was left to cool, and then the reaction liquid was added to dilute hydrochloric acid, extracted with ethyl acetate, and subjected to filtration using celite, followed by separating the layers, and then the organic layer was washed with a saturated aqueous solution of sodium hydrogencarbonate and a saturated aqueous solution of sodium chloride, and subsequently dried over magnesium sulfate. The solvent was evaporated under reduced pressure, and the resulting concentrate was purified by silica gel column chromatography, to obtain compound (2-IV) (1.0 g, 1.26 mmol, 25.2%).
   e) The compound (2-IV) (2.90 g, 3.65 mmol) was placed in a 200 mL autoclave, and was dissolved in tetrahydrofuran (60 mL). To the reaction system, 10% Pd-C (0.3 g) was added, followed by stirring at room temperature under a hydrogen pressure of 5 MPa for 3 hours. Then, the reaction liquid was filtrated, and the solvent was evaporated under reduced pressure, and then the resulting concentrate was washed by pouring methanol over it, to obtain compound (2-V) (2.02 g, 3.29 mmol, 90.1%) that was a dicarboxylic acid compound.
      ¹H-NMR data of the compound (2-V) are shown below.
      ¹H NMR (300 MHz, CDCl₃) δ 0.90 to 2.30 (m, 42H), 2.75 to 3.02 (m, 4H), 3.10 to 3.38 (m, 2H), 6.59 (s, 2H), 6.93 (s, 2H)
[3] Synthesis Example of Dicarboxylic Acid Compound Having a Structure Represented by Formula (IV) in which L¹ Represents a Sulfur Atom and Y Represents -OH 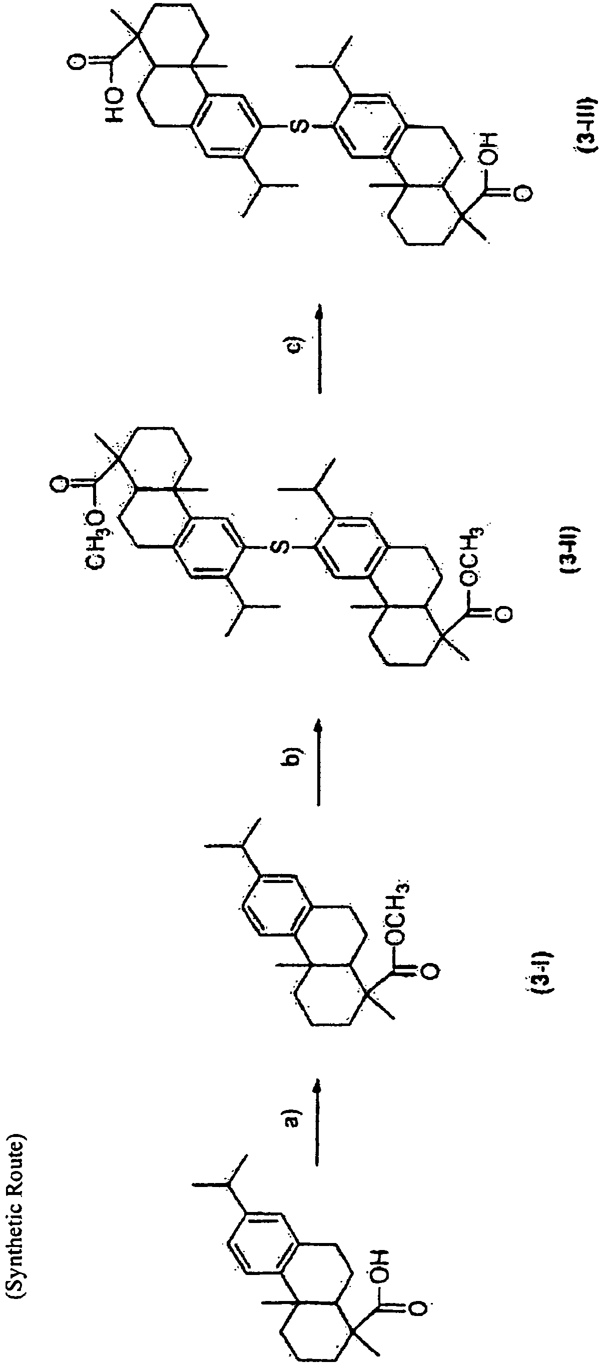
   a) Dehydroabietic acid (30.1 g, 100 mmol) was placed in a 300 mL three-necked flask and was dissolved in methylene chloride (100 mL). While blowing nitrogen into the flask, oxalyl chloride (10.3 mL, 120 mmol) was added thereto dropwise, followed by stirring at room temperature for one hour, and then the flask was placed in an ice bath and methanol (50 mL) was added thereto dropwise, followed by stirring for 3 hours. Then, the reaction solution was added to a saturated aqueous solution of sodium hydrogencarbonate, extracted with methylene chloride, followed by separating the layers, and then the organic layer was washed with a saturated aqueous solution of sodium chloride, and dried over magnesium sulfate. The solvent was evaporated under reduced pressure, and the resulting concentrate was washed by pouring methanol over it, to obtain compound (3-1) (26.7 g, 84.9 mmol, 84.9%).
   b) The compound (3-1) (12.6 g, 40.0 mmol) was placed in a 200 mL three-necked flask, and was dissolved in methylene chloride (30 mL). Then, disulfur dichloride (4.07 g, 30.4 mmol) was added thereto, and then the flask was placed in an ice bath and titanium tetrachloride (8.70 g, 45.9 mmol) was added thereto dropwise, followed by stirring at room temperature for 3 hours. Then, the reaction liquid was added to ice-water, extracted with ethyl acetate, followed by separating the layers, and then the organic layer was washed with a saturated aqueous solution of sodium chloride, and dried over magnesium sulfate. The solvent was evaporated under reduced pressure, and the resulting concentrate was purified by silica gel column chromatography, to obtain compound (3-II) (10.5 g, 15.9 mmol, 79.5%).
   c) The compound (3-II) (10.5 g, 15.9 mmol) was placed in a 500 mL three-necked flask equipped with a reflux tube, and was dissolved in a mixed solvent of ethanol (300 mL) and water (20 mL). Then, potassium hydroxide (20 g, 0.356 mol) was added thereto, and the resulting mixture was refluxed at 80°C for 18 hours. Then, the reaction liquid was left to cool, and then the reaction liquid was added to water, neutralized with dilute hydrochloric acid, extracted with ethyl acetate, followed by separating the layers, and then the organic layer was washed with a saturated aqueous solution of sodium chloride, and dried over magnesium sulfate. The solvent was evaporated under reduced pressure, and the resulting concentrate was washed by pouring methanol over it, to obtain compound (3-III) (7.25 g, 11.9 mmol, 74.8%) that was a dicarboxylic acid compound.
      ¹H-NMR data of the compound (3-III) are shown below.
      ¹H NMR (300 MHz, CDCl₃) δ 0.90 to 2.10 (m, 42H), 2.70 to 2.98 (m, 4H), 3.60 to 3.80 (m, 2H), 6.65 (s, 2H), 6.97 (s, 2H)
[4] Synthesis Example of Dicarboxylic Acid Compound Having a Structure Represented by Formula (IV) in which L¹ Represents Methylene and Y Represents -OH 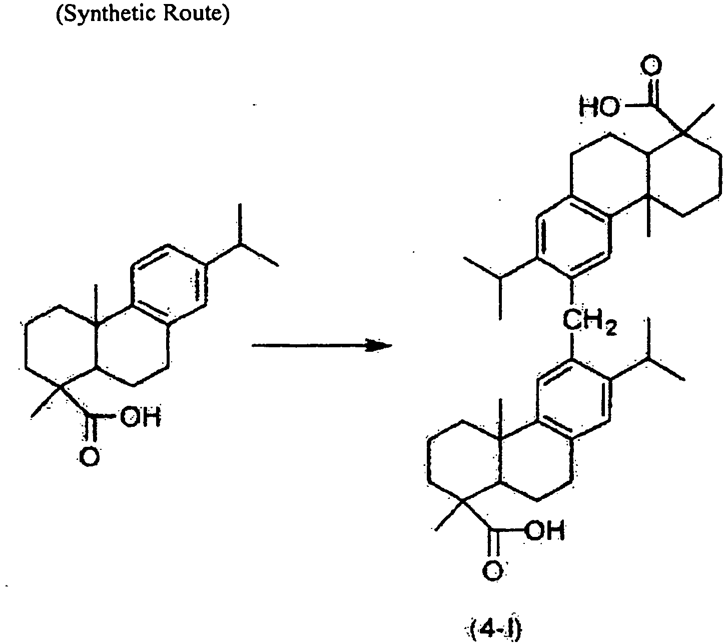
   Dehydroabietic acid (30.1 g, 0.100 mol) and a 36% aqueous formaldehyde solution (4.17 g, 0.0500 mol) were placed in a 500 mL three-necked flask and were dissolved in methylene chloride (100 mL). To the reaction system, sulfuric acid (20 mL) was added dropwise, followed by stirring at room temperature for 3 hours. Then, the reaction liquid was added to water, extracted with methylene chloride, followed by separating the layers, and then the organic layer was washed with a saturated aqueous solution of sodium chloride, and dried over magnesium sulfate. The solvent was evaporated under reduced pressure, and the resulting concentrate was purified by silica gel column chromatography, to obtain compound (4-1) (20.0 g, 0.0326 mol, 65.2%) that was a dicarboxylic acid compound.
   ¹H-NMR data of the compound (4-1) are shown below.
   ¹H NMR (300 MHz, CDCl₃) δ 0.90 to 2.30 (m, 42H), 2.76 to 2.98 (m, 4H), 3.00 to 3.18 (m, 2H), 3.96 (s, 2H), 6.68 (s, 2H), 6.95 (s, 2H), 11.10 (br-s, 2H),
[5] Synthesis Example of Self-Condensation Type Monomer 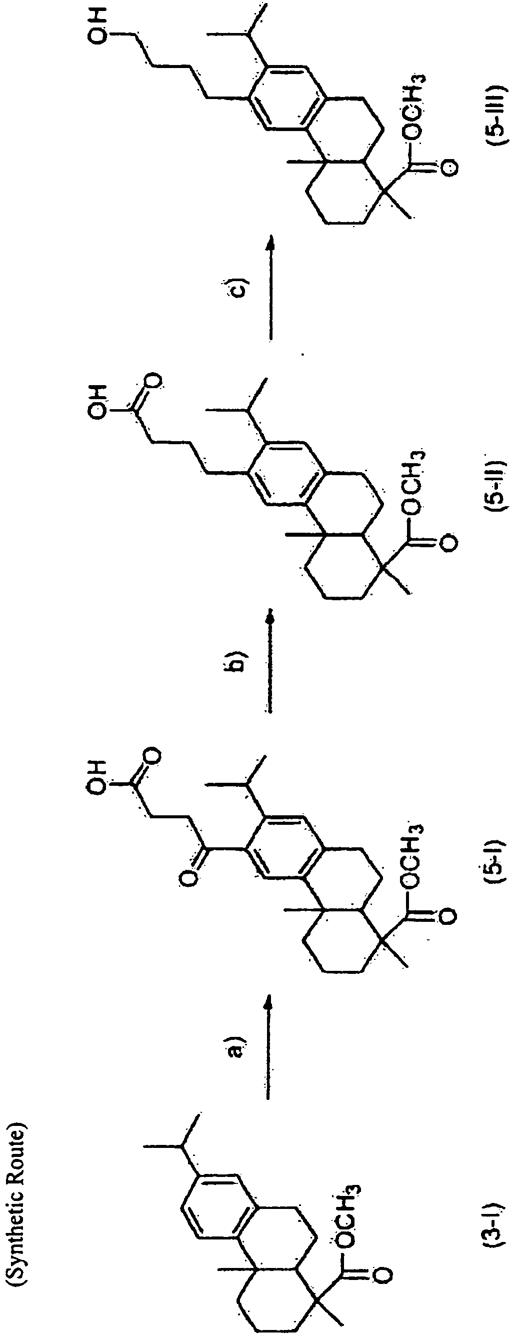
   a) Methyl dehydroabietate (the compound (3-1) obtained in [3] above) (44.0 g, 0.140 mol) and succinic anhydride (20.7 g, 0.207 mol) were placed in a 500 mL three-necked round-bottomed flask and were dissolved in methylene chloride (240 mL). To the reaction mixture, anhydrous aluminuim chloride (63.6 g, 0.477 mol) was added with small portion at a temperature of 10°C to 15°C. The mixture was stirred at room temperature for 3 hours, then, the reaction liquid was added to ice-water, extracted with methylene chloride, followed by separating the layers, and then the organic layer was washed with water, and dried over magnesium sulfate. The solvent was evaporated under reduced pressure, and then methanol was added to the resulting concentrate to conduct crystallization and washing by pouring, thereby obtaining compound (5-1) (49.6 g, 0.120 mol, 85.7%).
   b) The compound (5-I) (37.0 g, 89.3 mmol) and triethylsilane (31.2 g, 0.268 mol) were placed in a 300 mL three-necked round-bottomed flask equipped with a condenser tube and were suspended. The resulting suspension was heated to 40°C, and to the reaction system, trifluoroacetic acid (70.6 g, 0.619 mol) was added dropwise, followed by stirring at 65°C for 12 hours. Then, the reaction solution was left to cool, and then the reaction solution was added to ice, extracted with ethyl acetate, followed by separating the layers, the organic layer was washed with a saturated aqueous solution of sodium hydrogencarbonate, followed by adjusting the pH to 3, and then dried over magnesium sulfate. The solvent was evaporated under reduced pressure, and then n-hexane was added to the resulting concentrate to conduct crystallization and washing by pouring, thereby obtaining compound (5-II) (32.0 g, 79.9 mmol, 89.5%).
   c) The compound (5-II) (6.70 g, 16.7 mmol) was placed in a 100 mL three-necked round-bottomed flask and was dissolved in methylene chloride (20 mL). To the reaction mixture, oxalyl chloride (1.7 mL, 21.5 mmol) was added dropwise, followed by stirring at room temperature for 2 hours. Then, the reaction solution was concentrated, and the resulting concentrate was dissolved in tetrahydrofuran (30 mL). To the reaction system, sodium borohydride (1.3 g, 34.4 mmol) was added, followed by stirring at room temperature for 5 hours. Then, the reaction liquid was added to water, and 6 N hydrochloric acid was added thereto, and then the resulting mixture was extracted with ethyl acetate, followed by separating the layers, the organic layer was washed with water, and dried over magnesium sulfate. The solvent was evaporated under reduced pressure, and the resulting concentrate was purified by column chromatography, to obtain compound (5-III) (3.2 g, 8.28 mmol, 49.6%) that was a self-condensation type monomer.
      ¹H-NMR data of the compound (5-III) are shown below.
      ¹H NMR (300 MHz, CDCl₃) δ 1.07 to 1.98 (m, 23H), 2.12 to 2.39 (m, 2H), 2.50 to 2.70 (m, 2H), 2.77 to 2.95 (m, 2H), 3.00 to 3.19 (m, 1H), 3.60 to 3.75 (m, 2H), 3.65 (s, 3H), 6.89 (s, 1H), 6.97 (s, 1H)

### [Example 1]

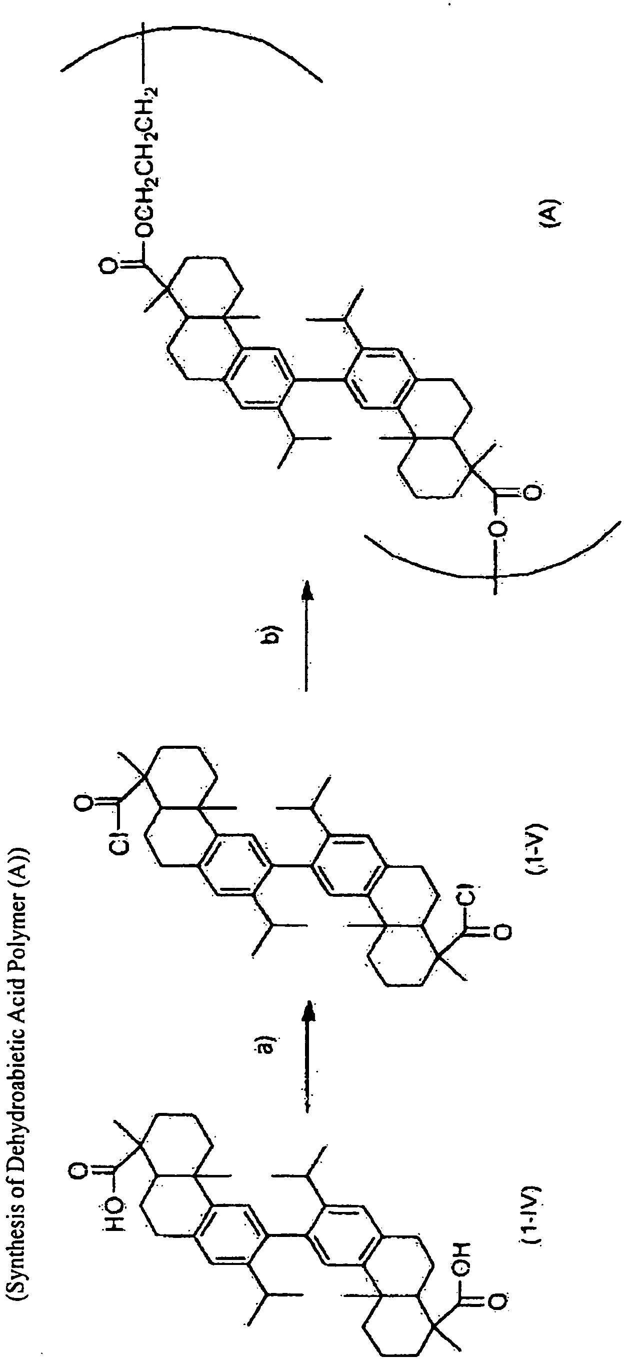

a) The compound (1-IV) (1.00 g, 1.67 mmol) was placed in a 50 mL three-necked flask and was suspended in methylene chloride (10 mL). Then, oxalyl chloride (0.3 mL, 3.50 mmol) was added thereto dropwise, and a catalytic amount of N,N-dimethylformamide was added, followed by stirring at room temperature for 2 hours. Thereafter, the reaction solution was concentrated, to obtain compound (1-V) (1.06 g, 1.67 mmol, q. y.).
b) The compound (1-V) (1.06 g, 1.67 mmol), that was a dicarboxylic acid chloride compound, and 1,3-propanediol (127 mg, 1.67 mmol) were placed in a 50 mL three-necked flask equipped with a nitrogen inlet tube, and were dissolved in 1,2-dichlorobenzene (1.5 mL). While blowing nitrogen into the flask, the resulting mixture was stirred and heated to 100°C, and reaction was carried out for one hour, subsequently at 170°C for 5 hours, and further, at 200°C for 5 hours. The reaction solution was left to cool, then the reaction product was dissolved in dichloromethane (5 mL), and poured into 2-propanol (200 mL), and then the precipitates separated were filtered off. The precipitates were dissolved in tetrahydrofuran (5 mL), and insoluble matters were removed, and then the resulting solution was poured into 2-propanol (200 mL), and the reprecipitated polymer was filtered off, washed with 2-propanol, and dried, to obtain a powdery polyester (0.98 g), which was designated as dehydroabietic acid polymer (A).

The weight average molecular weight of the dehydroabietic acid polymer (A) as measured by GPC was 12,000. Further, concerning the thermal physical properties of the dehydroabietic acid polymer (A), the glass transition temperature Tg as measured by DSC at a temperature raising rate of 10 °C/min was 200°C.
¹H-NMR data of the dehydroabietic acid polymer (A) are shown below.
¹H NMR (300 MHz, CDCl₃) δ 0.90 to 2.40 (m, 44H), 2.49 to 2.77 (m, 2H), 2.81 to 3.09 (m, 4H), 3.39 to 4.37 (m, 4H), 6.88 to 6.98 (m, 2H), 6.98 (s, 2H)

### [Example 2]

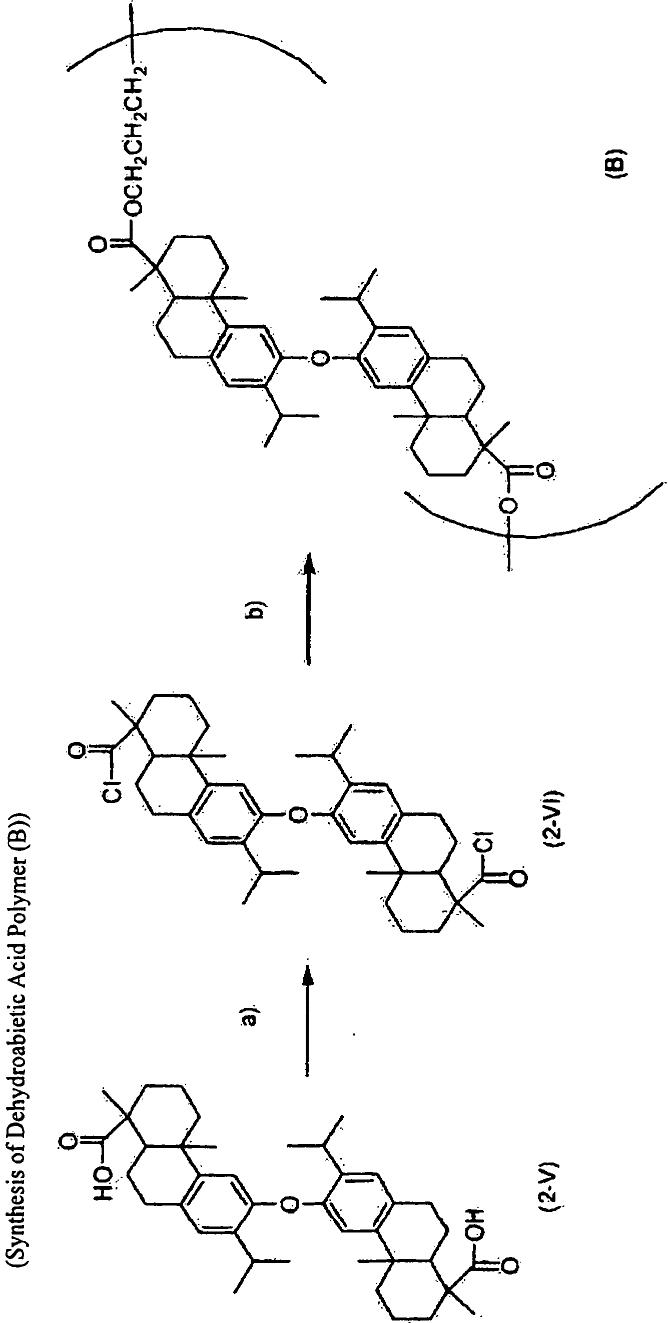

Acid chloride (2-VI) (1.96 g, 3.01 mmol) was obtained in a manner substantially similar to that in Example 1, except that the compound (2-V) (1.85 g, 3.01 mmol) was used as the dicarboxylic acid compound, and thereafter, the obtained acid chloride and 1,3-propanediol (229 mg, 3.01 mmol) were allowed to undergo polycondensation reaction and subjected to treatment, to obtain a polyester (1.45 g), which was designated as dehydroabietic acid polymer (B).

The weight average molecular weight of the dehydroabietic acid polymer (B) as measured by GPC was 10,700. Further, concerning the thermal physical properties of the dehydroabietic acid polymer (B), the glass transition temperature Tg as measured by DSC at a temperature raising rate of 10 °C/min was 138°C.
¹H-NMR data of the dehydroabietic acid polymer (B) are shown below.
¹H NMR (300 MHz, CDCl₃) δ 0.90 to 2.30 (m, 44H), 2.75 to 3.02 (m, 4H), 3.10 to 3.38 (m, 2H), 4.00 to 4.30 (m, 4H), 6.59 (s, 2H), 6.93 (s, 2H)

### [Example 3]

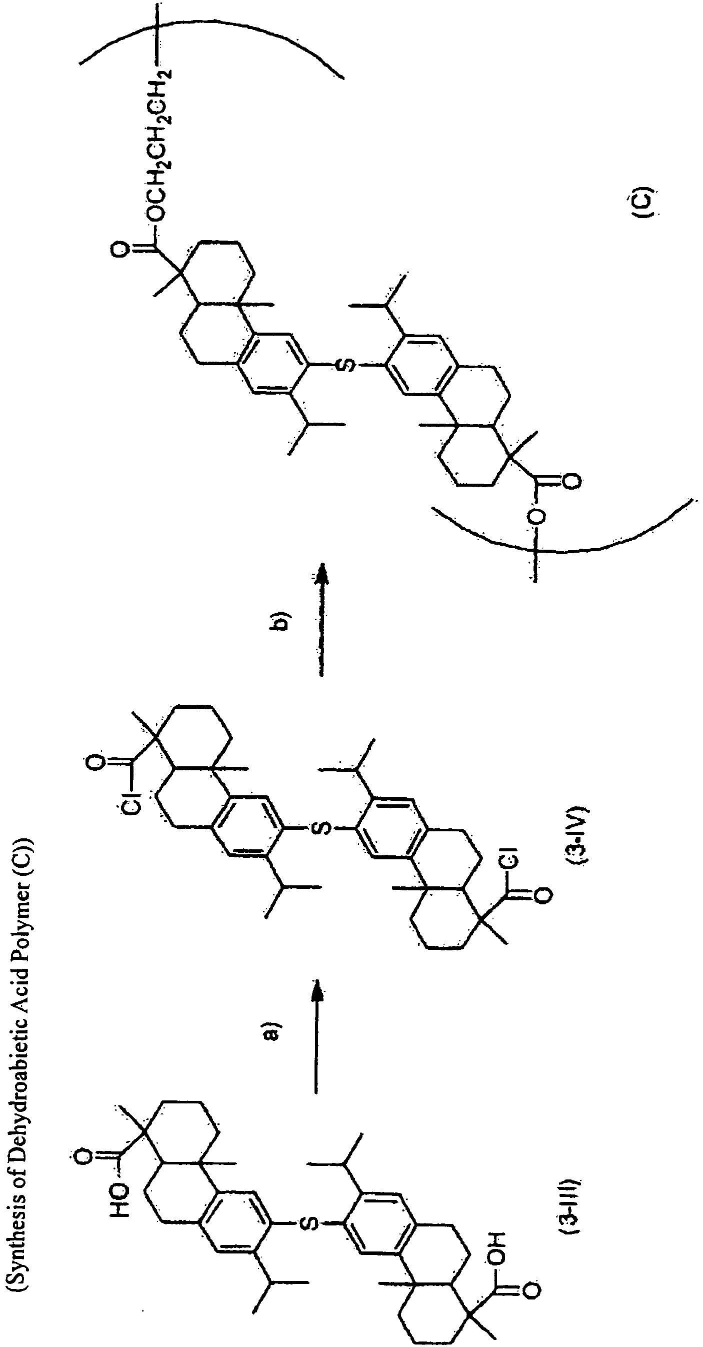

Acid chloride (3-IV) was obtained in a manner substantially similar to that in Example 1, except that the compound (3-III) (3.15 g, 4.99 mmol) was used as the dicarboxylic acid compound, and thereafter, the compound (3-IV) (3.33 g, 4.99 mmol) and 1,3-propanediol (380 mg, 4.99 mmol) were placed in a 50 mL three-necked flask, and were dissolved in dichloromethane (10 mL). Absolute pyridine (10 mL) was added thereto, and while blowing nitrogen into the flask, the resulting mixture was stirred at room temperature for one hour, then the temperature of the mixture was raised to 40°C, and reaction was carried out for 2 hours and subsequently at 60°C for 3 hours. The reaction solution was left to cool, then the reaction product was poured into methanol (100 mL), and the precipitates separated were filtered off. The precipitates were dissolved in tetrahydrofuran (30 mL), and insoluble matters were removed, and then the resulting solution was poured into methanol (100 mL), to perform reprecipitation. The resulting polymer was filtered off, washed with methanol, and dried, to obtain a powdery polyester (1.85 g), which was designated as dehydroabietic acid polymer (C).

The weight average molecular weight of the dehydroabietic acid polymer (C) as measured by GPC was 6,600. Further, concerning the thermal physical properties of the dehydroabietic acid polymer (C), the glass transition temperature Tg as measured by DSC at a temperature raising rate of 10 °C/min was 105°C.
¹H-NMR data of the dehydroabietic acid polymer (C) are shown below.
¹H NMR (300 MHz, CDCl₃) δ 0.90 to 2.29 (m, 44H), 2.70 to 2.98 (m, 4H), 3.19 to 3.49 (m, 2H), 3.98 to 4.27 (m, 4H), 6.85 (s, 2H), 7.47(s, 2H)

### [Example 4]

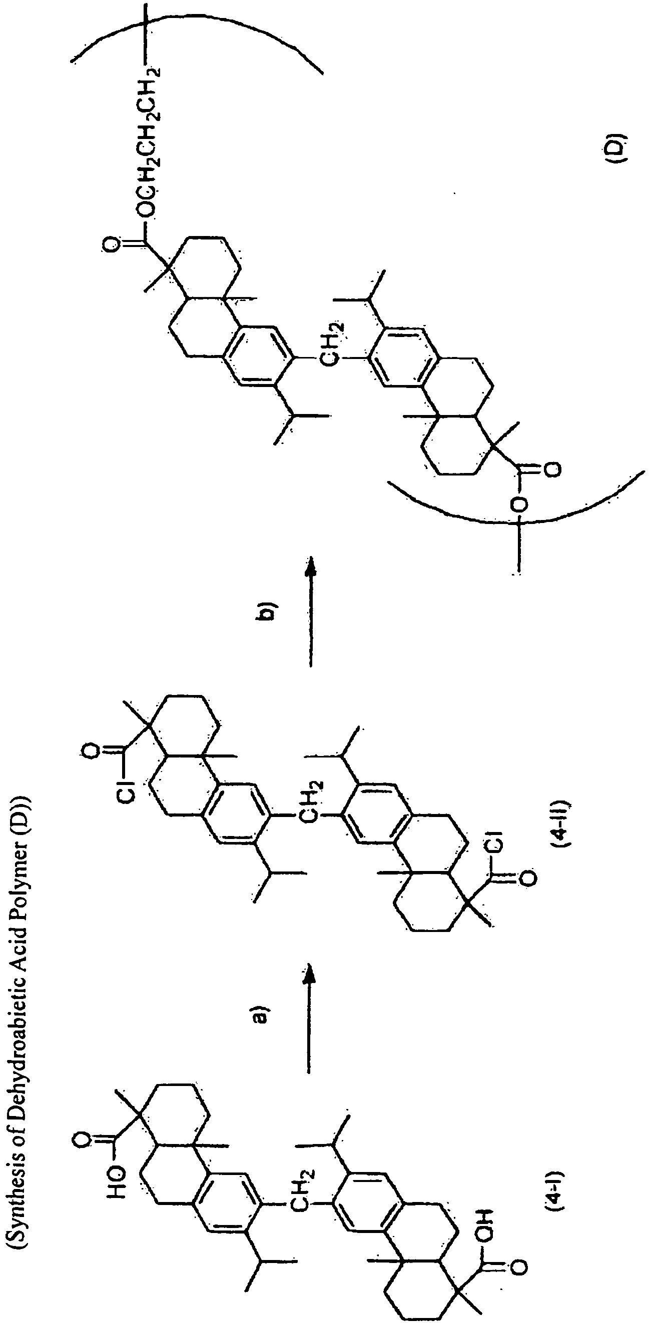

Acid chloride (4-II) was obtained in a manner substantially similar to that in Example 1, except that the compound (4-1) (24.5 g, 40.0 mmol) was used as the dicarboxylic acid compound, and thereafter, the compound (4-II) (26.0 g, 40.0 mmol) and 1,3-propanediol (3.04 g, 40.0 mmol) were placed in a 200 mL three-necked flask equipped with a nitrogen inlet tube, and were dissolved in dichloromethane (20 mL). Absolute pyridine (50 mL) was added thereto dropwise, and while blowing nitrogen into the flask, the resulting mixture was stirred at room temperature for one hour, then the temperature of the mixture was raised to 50°C, and reaction was carried out for one hour, subsequently at 100°C for 3 hours, and further, at 120°C for 5 hours. The reaction liquid was left to cool, then the reaction product was poured into methanol (1 L), and then the precipitates separated were filtered off. The precipitates were dissolved in tetrahydrofuran (50 mL), and insoluble matters were removed, and then the resulting solution was poured into methanol (1 L), and the reprecipitated polymer was filtered off, washed with methanol, and dried, to obtain a powdery polyester (21.5 g), which was designated as dehydroabietic acid polymer (D).

The weight average molecular weight of the dehydroabietic acid polymer (D) as measured by GPC was 11,600. Further, concerning the thermal physical properties of the dehydroabietic acid polymer (D), the glass transition temperature Tg as measured by DSC at a temperature raising rate of 10 °C/min was 125°C.
¹H-NMR data of the dehydroabietic acid polymer (D) are shown below.
¹H NMR (300 MHz, CDCl₃) δ 0.90 to 2.30 (m, 44H), 2.76 to 2.98 (m, 4H), 3.00 to 3.18 (m, 2H), 3.96 (s, 2H), 4.02 to 4.25 (m, 4H), 6.68 (s, 2H), 6.95(s, 2H)

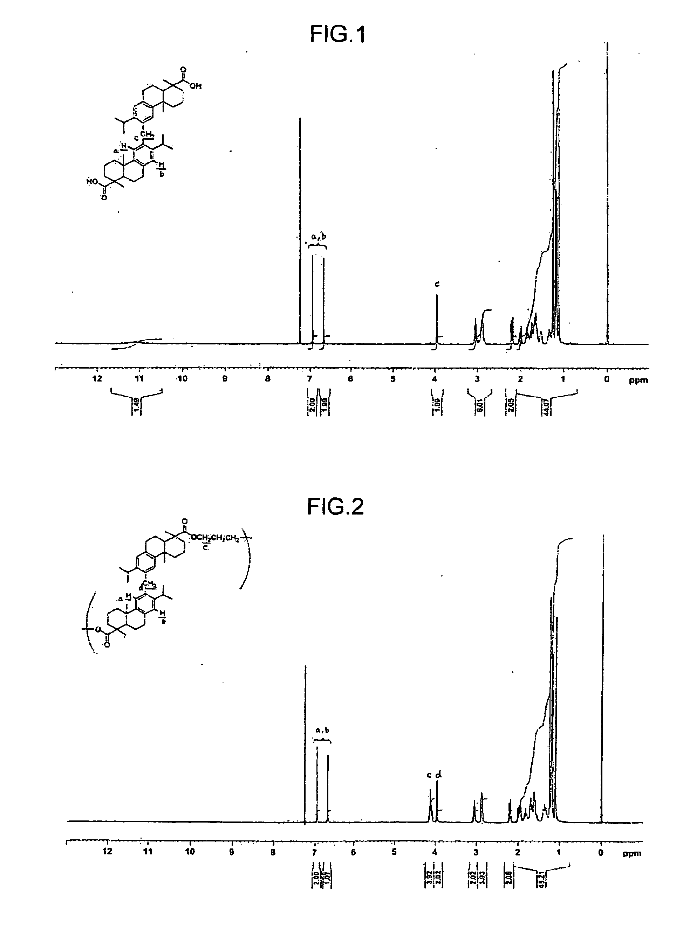

Fig. 1 shows a ¹H-NMR spectrum of compound (4-1) used in the Example. Further, Fig. 2 shows a ¹H-NMR spectrum of the obtained dehydroabietic acid polymer (D).

### [Example 5]

### (Synthesis of Dehydroabietic Acid Polymer (E))

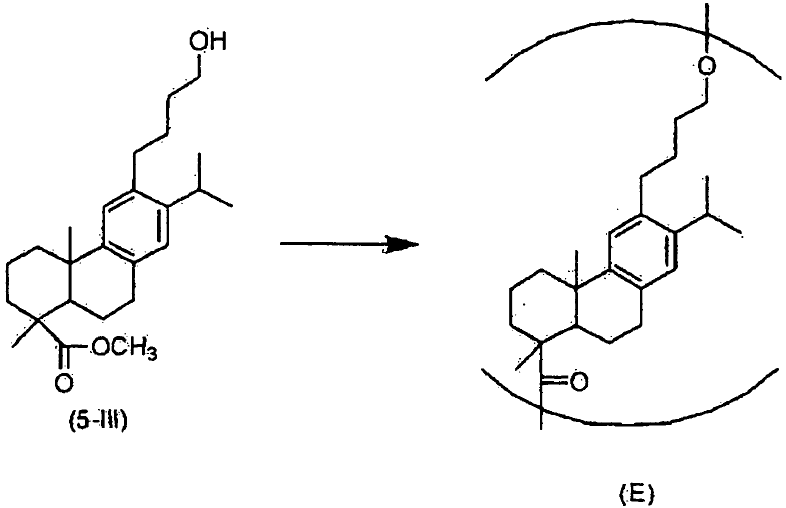

The compound (5-III) (2.0 g, 5.17 mmol) was placed in a 50 mL three-necked round-bottomed flask equipped with a nitrogen inlet tube, and tetraethyl orthotitanate (100 mg, 0.438 mmol) was added thereto. Under a reduced pressure of 200 mmHg to 250 mmHg, while letting dry nitrogen flow gently, the temperature of the mixture was gradually raised to 200°C, the mixture was heated for 2 hours, and the generated methanol was distilled off. Further, the mixture was heated at 220°C for 2 hours, and then at 250°C for 2 hours. The viscous liquid was left to cool, and then methanol was added thereto, the precipitates separated were filtered off, and the precipitates were washed with methanol. The resulting precipitates were dried and ground, to obtain a powdery polyester (1.80 g), which was designated as dehydroabietic acid polymer (E).

The weight average molecular weight of the dehydroabietic acid polymer (E) as measured by GPC was 9,700. Further, concerning the thermal physical properties of the dehydroabietic acid polymer (E), the glass transition temperature Tg as measured by DSC at a temperature raising rate of 10 °C/min was 102°C.
¹H-NMR data of the dehydroabietic acid polymer (E) are shown below.
¹H NMR (300 MHz, CDCl₃) δ 1.07 to 1.98 (m, 23H), 2.12 to 2.41 (m, 2H), 2.45 to 2.71 (m, 2H),2.72 to 2.95 (m, 2H), 2.96 to 3.15(m, 1H), 3.92 to 4.25 (m, 2H), 6.88 (s, 1H), 6.96(s, 1H)

### [Evaluation]

Using the dehydroabietic acid polymers (A) to (E) obtained in Examples 1 to 5 and commercially available PC (polycarbonate), PET (polyethylene terephthalate), and PLA (polylactic acid) as comparative polymers in Comparative Examples 1 to 3, physical properties, that is, glass transition temperature Tg (°C), coefficient of water absorption (%), and degree of hydrolysis of the polymers were compared and evaluated. The evaluation results are shown in Table 1 below.

Details on the PC, PET, and PLA used as the comparative polymers in Comparative Examples 1 to 3 are as follows.
PC: polycarbonate manufactured by Teijin Chemicals Ltd., trade name: PANLIGHT L-1225Y, Tg: 150°C
PET: polyethylene terephthalate manufactured by Sigma-Aldrich Corporation, trade name: POLY(ETHYLENE TEREPHTHALATE) GRANULAR, Tg: 67°C
PLA: polylactic acid manufactured by Mitsui Chemicals, Inc., trade name: LACEA H-140, Tg: from 57°C to 60°C

### <Coefficient of Water Absorption (%)>

The coefficient of water absorption was measured as follows.

Each (1 g) of the dehydroabietic acid polymers (A) to (E) obtained in Examples 1 to 5 and the commercially available PC, PET, and PLA in Comparative Examples 1 to 3 was heat pressed (at a temperature of from 160°C to 250°C) to prepare a film having a thickness of 200 µm. The obtained film was dipped in water at 23°C for 24 hours, and then water droplets residing on the surfaces were wiped out well, and the weight of the film was measured quickly. The coefficient of water absorption was calculated according to the following equation.

Coefficient of Water Absorption (%) = (Weight of Film After Dipping in Water-Weight of Film Before Dipping in Water)/Weight of Film Before Dipping in Water

### <Degree of Hydrolysis>

The degree of hydrolysis was measured as follows.

Each (1 g) of the dehydroabietic acid polymers (A) to (E) obtained in Examples 1 to 5 and the commercially available PC, PET, and PLA used as comparative polymers in Comparative Examples 1 to 3 was dissolved in THF (tetrahydrofuran) (30 mL) and in 1,2-dichloroethane (30 mL), respectively, and 1 N NaOH aqueous solution (10 mL) was added to the THF solution, and on the other hand, sulfuric acid (0.1 mL) was added to the 1,2-dichloroethane solution, followed by stirring for 24 hours. The solution that had been stirred was poured in water, and the weight average molecular weight of the precipitates separated was measured by GPC.

With regard to the dehydroabietic acid polymers and the comparative polymers, the ratio of the weight average molecular weight after hydrolysis relative to the weight average molecular weight before hydrolysis was designated as the degree of hydrolysis.

**TABLE 1**

| | Dehydroabietic Acid Polymer or Comparative Polymer | Glass Transition Temperature Tg (°C) | Water Absorption (%) | Degree of Hydrolysis | |
|---|---|---|---|---|---|
| | | | | Acid | Alkali |
| Example 1 | (A) | 200 | 0.18 | 0.93 | 0.97 |
| Example 2 | (B) | 138 | 0.2 | 0.91 | 0.96 |
| Example 3 | (C) | 105 | 0.21 | 0.9 | 0.94 |
| Example 4 | (D) | 125 | 0.17 | 0.91 | 0.97 |
| Example 5 | (E) | 102 | 0.19 | 0.92 | 0.97 |
| Comparative Example 1 | PC | 150 | 0.25 | 0.7 | 0.03 |
| Comparative Example 2 | PET | 67 | 0.52 | 0.75 | 0.08 |
| Comparative Example 3 | PLA | 57-60 | 0.34 | 0.13 | 0.02 |

As shown in Table 1, it was understood that the dehydroabietic acid polymers (A) to (E) (polyester polymers) obtained in Examples 1 to 5 exhibited improved heat resistance and improved moisture and water resistance, as compared to the PET and PLA. Further, it was also understood that, as compared to the PC, the dehydroabietic acid polymers (A) to (E) exhibited improved moisture and water resistance.

A multi-purpose test piece according to JIS K 7139 was prepared by injection molding using the dehydroabietic acid polymer of each of the Examples. All of the dehydroabietic acid polymers of the Examples exhibited excellent moldability, and it was confirmed that the obtained test pieces were substances superior in strength usable as a member for electronic equipments.

## Claims

1. A dehydroabietic acid polymer having a weight average molecular weight of from 5,000 to 500,000 and comprising a repeating unit represented by the following Formula (II): 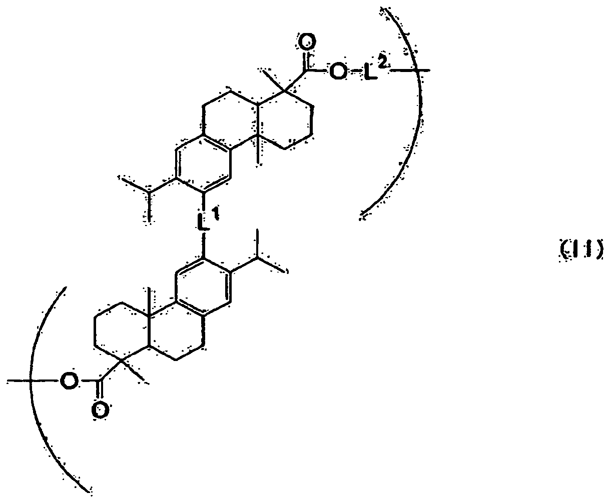 wherein L¹ represents a single bond, -O-, -S- or -CH₂-; and L² represents an alkylene group or an arylene group.

2. A dehydroabietic acid polymer having a weight average molecular weight of from 5,000 to 500,000 and comprising a repeating unit represented by the following Formula (III): 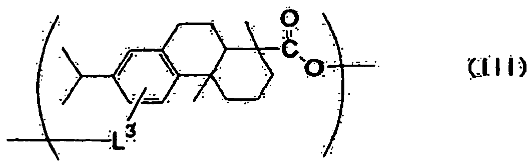 wherein L³ represents a single bond, -(CH₂)₄-, -CO(CH₂)₃-, -(CH₂)₃CO₂-C₆H₄-C(CH₃)₂-C₆H₄- or -CO(CH₂)₂CO₂-C₆H₄-C(CH₃)₂-C₆H₄-.

3. A composite material comprising the dehydroabietic acid polymer as defined in Claim 1 or Claim 2.

4. A dehydroabietic acid derivative comprising a compound represented by the following Formula (IV): 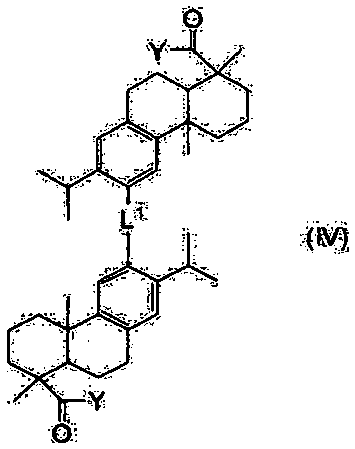 wherein L¹ represents a single bond, -O-, -S- or -CH₂-; Y represents -OH, -OR, -OCOR, -OCOOR, or -OSO₂R; and R represents an alkyl group or an aryl group.

## Patentansprüche

1. Dehydroabietinsäurepolymer, das ein gewichtsgemitteltes Molekulargewicht von 5.000 bis 500.000 aufweist und eine durch die folgende Formel (II) dargestellte Wiederholungseinheit umfasst: 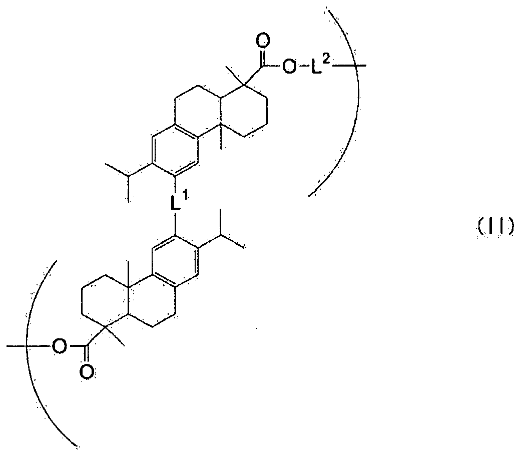 worin L¹ eine Einfachbindung, -O-, -S- oder -CH₂-darstellt; und L² eine Alkylengruppe oder eine Arylengruppe darstellt.

2. Dehydroabietinsäurepolymer, das ein gewichtsgemitteltes Molekulargewicht von 5.000 bis 500.000 aufweist und eine durch die folgende Formel (III) dargestellte Wiederholungseinheit umfasst: 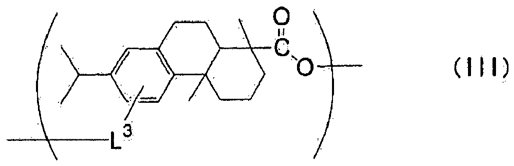 worin L³ eine Einfachbindung, -(CH₂)₄-, -CO(CH₂)₃-, -(CH₂)₃CO₂-C₆H₄-C(CH₃)₂-C₆H₄- oder -CO(CH₂)₂CO₂-C₆H₄-C(CH₃)₂-C₆H₄- darstellt.

3. Kompositmaterial, welches das Dehydroabietinsäurepolymer gemäß Anspruch 1 oder Anspruch 2 umfasst.

4. Dehydroabietinsäurederivat, umfassend eine durch die folgende Formel (IV) dargestellte Verbindung: 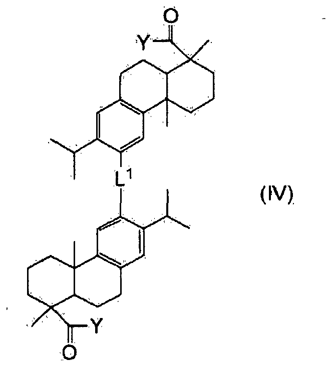 worin L¹ eine Einfachbindung, -O-, -S- oder -CH₂-darstellt; Y -OH, -OR, -OCOR, -OCOOR oder -OSO₂R darstellt; und R eine Alkylgruppe oder eine Arylgruppe darstellt.

## Revendications

1. Polymère d'acide déshydroabiétique ayant un poids moléculaire moyen en poids de 5 000 à 500 000 et comprenant un motif répété représenté par la Formule (II) suivante : 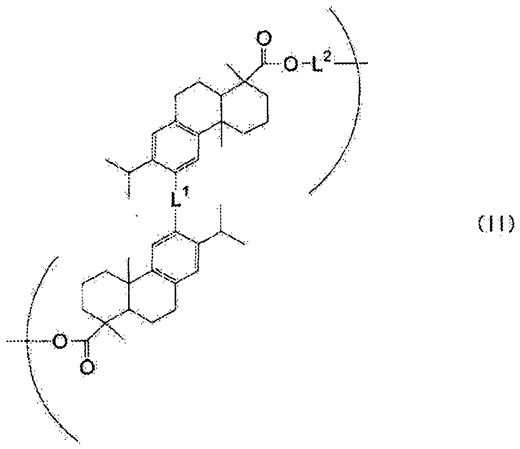 dans laquelle L¹ représente une liaison simple, -O-, -S- ou -CH₂- ; et L² représente un groupe alkylène ou un groupe arylène.

2. Polymère d'acide déshydroabiétique ayant un poids moléculaire moyen en poids de 5 000 à 500 000 et comprenant un motif répété représenté par la Formule (III) suivante : 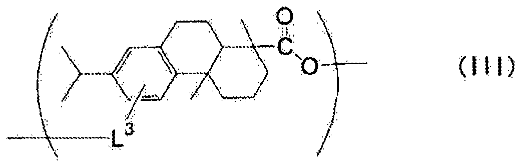 dans laquelle L³ représente une liaison simple, -(CH₂)₄-, -CO(CH₂)₃-, -(CH₂)₃CO₂-C₆H₄-C(CH₃)₂-C₆H₄- ou -CO(CH₂)₂CO₂-C₆H₄-C(CH₃)₂-C₆H₄-.

3. Matériau composite comprenant le polymère d'acide déshydroabiétique tel que défini dans la revendication 1 ou la revendication 2.

4. Dérivé d'acide déshydroabiétique comprenant un composé représenté par la Formule (IV) suivante : 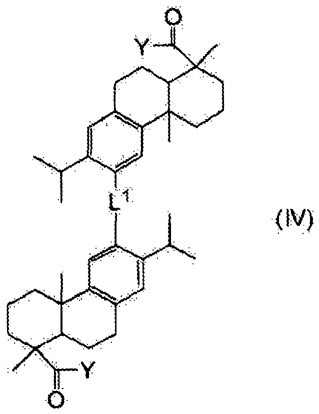 dans laquelle L¹ représente une liaison simple, -O-, -S- ou -CH₂- ; Y représente -OH, -OR, -OCOR, -OCOOR, ou -OSO₂R ; et R représente un groupe alkyle ou un groupe aryle.
